(19) Europäisches Patentamt
European Patent Office
Office européen des brevets

(11) EP 1 351 940 B1

(12) FASCICULE DE BREVET EUROPEEN

(45) Date de publication et mention de la délivrance du brevet: **25.07.2007 Bulletin 2007/30**

(21) Numéro de dépôt: **02700323.5**

(22) Date de dépôt: **15.01.2002**

(51) Int Cl.:
***C07D 215/04*** *(2006.01)* ***A61K 31/47*** *(2006.01)*
***A61P 33/02*** *(2006.01)* ***C07D 215/02*** *(2006.01)*
***C07D 215/12*** *(2006.01)* ***C07D 215/24*** *(2006.01)*
***C07D 405/06*** *(2006.01)* ***C07F 7/08*** *(2006.01)*

(86) Numéro de dépôt international: **PCT/FR2002/000140**

(87) Numéro de publication internationale: **WO 2002/057238 (25.07.2002 Gazette 2002/30)**

# (54) QUINOLEINES SUBSTITUEES POUR LE TRAITEMENT DE CO-INFECTIONS A PROTOZOAIRES ET A RETROVIRUS

SUBSTITUIERTE CHINOLINE ZUR BEHANDLUNG VON DURCH PROTOZOEN UND RETROVIREN VERURSACHTEN CO-INFEKTIONEN

SUBSTITUTED QUINOLINES FOR THE TREATMENT OF PROTOZOAN AND RETROVIRUS CO-INFECTIONS

(84) Etats contractants désignés:
**AT BE CH CY DE DK ES FI FR GB GR IE IT LI LU MC NL PT SE TR**

(30) Priorité: **17.01.2001 FR 0100580**

(43) Date de publication de la demande: **15.10.2003 Bulletin 2003/42**

(73) Titulaires:
- **Institut de Recherche pour le Développement ( IRD) 75010 Paris (FR)**
- **CENTRE NATIONAL DE LA RECHERCHE SCIENTIFIQUE (CNRS) 75016 Paris (FR)**

(72) Inventeurs:
- **FAKHFAKH, Mohamed 3001 Sfax (TN)**
- **FIGADERE, Bruno F-91530 SAINT-CHERON (FR)**
- **FOURNET, Alain F-40290 OSSAGES (FR)**
- **FRANCK, Xavier F-94550 CHEVILLY-LARUE (FR)**
- **HOCQUEMILLER, Reynald F-91470 LIMOURS (FR)**
- **PRINA, Eric F-78340 LES-CLAYES-SOUS-BOIS (FR)**

(74) Mandataire: **Corizzi, Valérie et al Cabinet ORES, 36, rue de Saint Pétersbourg 75008 Paris (FR)**

(56) Documents cités:
**WO-A-93/07125** **FR-A- 2 761 687**

- **CHEMICAL ABSTRACTS, vol. 121, no. 7, 15 août 1994 (1994-08-15) Columbus, Ohio, US; abstract no. 73107s, FOURNET, A: ET AL.: "The activity of 2-substituted quinoline alkaloids in BALB/c mice infected with Leishmania donovani." XP002201926 & J. ANTIMICROB: CHEMOTHER., vol. 33, no. 3, - 1994 pages 537-544,**
- **MOHAMED A. FAKHFAKH ET AL.: "Expeditious preparation of 2-substituted quinolines" TETRAHEDRON LETTERS., vol. 42, no. 23, - 4 juin 2001 (2001-06-04) pages 3847-3850, XP002201925 ELSEVIER SCIENCE PUBLISHERS, AMSTERDAM., NL ISSN: 0040-4039**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 351 940 B1

Printed by Jouve, 75001 PARIS (FR)

## Description

L'invention se rapporte à des quinoléines substituées pour le traitement des co-infections à protozoaires et à rétrovirus.

Les zones géographiques d'endémie des maladies dues aux protozoaires (leishmanioses, trypanosomiases, paludisme, ...) sont également des zones de haute prévalence des maladies rétrovirales, et notamment du syndrome de l'immunodéficience acquise ou SIDA.

De ce fait, depuis plusieurs années, on assiste à l'émergence de co-infections à protozoaires et à rétrovirus du type *Leishmania*/VIH-1*, Plasmodium*/VIH ou encore *Pneumocystis carinii*/VIH dont le nombre ne cesse d'augmenter, qui présentent une extrême gravité et réduisent fortement l'espérance de vie des personnes qui en sont atteintes. Ainsi, par exemple, la leishmaniose viscérale ou kala-azar, qui est induite par *Leishmania donovani* et *Leishmania infantum* et qui représente la forme la plus sévère des leishmanioses, est considérée comme un facteur particulièrement aggravant de l'évolution du SIDA (OMS, 1997, Weekly Epidemiol. Rec., 72 : 49-54).

On sait que certains protozoaires comme les leishmanies, les trypanosomes et les toxoplasmes, et le VIH possèdent la faculté d'infecter et de se multiplier dans des cellules hôtes particulières telles que les macrophages, les monocytes et les cellules dendritiques, qui jouent un rôle primordial dans la mise en place et le développement des réponses immunitaires vis-à-vis des agents pathogènes.

On sait également que la co-infection de ces cellules par des agents pathogènes différents a pour conséquence d'augmenter l'affaiblissement du système immunitaire et de favoriser la multiplication et la dissémination de ces agents pathogènes dans l'organisme (Wolday et al., Parasitology Today, 1999, 15 : 182-187).

Ainsi, par exemple, des études réalisées *in vitro* sur des cellules d'origine monocytaire ont montré que la présence de leishmanies dans ce type de cellules peut, lorsque celles-ci sont ultérieurement infectées par le VIH, induire et activer la réplication de ce dernier (Bernier et al., 1995, J. Virol., 69 : 7282-7275). A l'inverse, la présence du VIH est susceptible d'accroître la croissance intracellulaire des leishmanies lorsque les cellules sont infectées secondairement par ces protozoaires (Wolday et al., 1998, Scand J. Infect. Dis., 30 : 29-34). Par ailleurs, un pourcentage important de patients présentant une co-infection *Leishmania*/HIV montrent une multiplication et une dissémination élevées des leishmanies. Les mécanismes immunopathologiques par lesquels les leishmanies et le VIH interagissent sur le système immunitaire et se renforcent mutuellement n'ont pas encore été élucidés. De nombreuses hypothèses ont été avancées mais aucune d'entre elles n'a été réellement confirmée à ce jour (Wolday et *al.,* 1999, ibid).

Les co-infections à protozoaires et à rétrovirus posent des difficultés particulières en termes de thérapie.

Si l'on prend par exemple le cas des co-infections *Leishmania*/VIH, les traitements antileshmaniens ne sont pas toujours efficaces et les échecs et rechutes liés à des phénomènes de résistance ou de toxicité des produits sont courants. Ainsi, une étude réalisée dans le sud-ouest de l'Europe a montré que 52% de patients porteurs d'une co-infection *Leishmania*/VIH et traités par des antimoniés pentavalents comme l'antimoniate de méglumine (Glucantime®) - qui représente le traitement de première intention de la leishmaniose - font une à quatre rechutes dans un délai d'un mois à trois ans (source OMS).

Au surplus, tous les antileishmaniens disponibles à ce jour (antimoniate de méglumine, pentamidine, amphotéricine B) doivent être administrés par voie parentérale, ce qui rend leur utilisation coûteuse, peu compatible avec une absence de structures hospitalières et, donc, bien souvent inaccessible à la majeure partie des populations des régions touchées par les co-infections *Leishmania*/VIH.

Quant aux traitements antirétroviraux, s'il n'est pas contestable que leur administration en trithérapie (association de deux inhibiteurs de la transcriptase inverse et d'un inhibiteur de la protéase du VIH) améliore le pronostic des co-infections *Leishmania*/VIH, on sait qu'ils induisent des mutations virales qui sont responsables de l'apparition de résistances et, à terme, d'un phénomène d'échappement thérapeutique entraînant une reprise de l'infection. De plus, les résistances induites étant le plus souvent croisées entre les différents antirétroviraux, notamment entre les inhibiteurs de la protéase, les substitutions thérapeutiques sont très restreintes. L'utilisation de la trithérapie présente, par ailleurs, l'inconvénient majeur d'être d'un coût très élevé et d'être, elle aussi, inaccessible à la plus grande part des populations des régions touchées par les co-infections *Leishmania*/VIH.

Dans la demande Internationale PCT WO 93/07125, les Inventeurs ont mis en évidence que des quinoléines substituées au niveau de l'atome de carbone situé en position 2 du cycle quinoléique par un groupe n-propyle, hydroxypropyle, n-propényle, *trans*-époxypropyle ou styryle, sont actives aussi bien sur les leishmanies responsables des leishmanioses cutanée et cutanéo-muqueuse *(Leishmania amazonensis, Leishmania venezuelensis,* ...) que sur celles responsables de la leishmaniose viscérale, lorsqu'elles sont administrées à des souris et ce, même par voie orale.

Par ailleurs, MEKOUAR et *al.* ont montré, dans la Demande Internationale PCT WO 98/45269 ainsi que dans un article publié dans Journal of Medicinal Chemistry (2000, 43 : 1533-1540), que des 2-styrylquinoléines sont capables d'inhiber l'intégrase du VIH *in vitro* et la réplication de ce virus dans des cellules d'une lignée lymphocytaire (CEM) préalablement infectées.

Ces auteurs indiquent, toutefois, dans leur article que l'activité inhibitrice de quinoléines substituées vis-à-vis

de l'intégrase du VIH nécessite, non seulement la présence d'un cycle aromatique ancillaire - qui est représenté en l'espèce par le groupe phényle du radical styryle -, mais également celle d'un groupe carboxyle et d'un groupe hydroxyle respectivement en C-7 et C-8 du cycle quinoléique. L'activité inhibitrice sur la réplication intracellulaire du VIH exigerait, quant à elle, la présence supplémentaire d'une paire de substituants situés en *ortho* sur le cycle aromatique ancillaire, à savoir un groupe hydroxyle ou méthoxy en C-3' et un groupe hydroxyle en C-4'.

Or, poursuivant leurs travaux sur les quinoléines substituées, les Inventeurs ont constaté que de manière surprenante, certaines de ces quinoléines, bien que ne répondant pas aux critères structurels énoncés par les auteurs précédents, présentent à la fois une activité anti-protozoaire et une aptitude à inhiber la réplication intracellulaire de rétrovirus, et notamment du VIH, et sont en conséquence propres à constituer une thérapeutique de choix pour les co-infections à protozoaires et à rétrovirus.

La présente Invention a donc pour objet l'utilisation d'au moins une quinoléine répondant à la formule générale (I) :

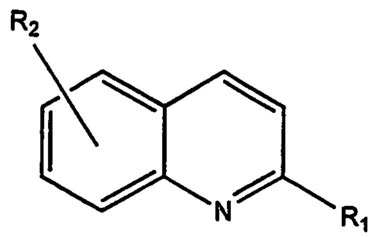

(I)

dans laquelle :

- $R_1$ représente :

  - un atome d'hydrogène, un groupe alkyle en $C_1$ à $C_{15}$, un groupe alkényle en $C_2$ à $C_{15}$, un groupe alkynyle en $C_2$ à $C_{15,}$ un groupe formyle ou un groupe hétéroaryle, ce dernier étant éventuellement substitué par un ou plusieurs groupes hydroxyle ; ou bien
  - un groupe alkyle en $C_1$ à $C_{15}$ ou alkényle en $C_2$ à $C_7$ portant au moins un substituant choisi parmi l'oxygène, les halogènes et les groupes hydroxyle, formyle, carboxyle, aryloxycarbonyle, alkyloxycarbonyle en $C_2$ à $C_8$, alkényloxycarbonyle en $C_3$ à $C_9$, nitrile, amine, alcoxy en $C_1$ à $C_7$, phénoxy, cycloalkyle en $C_3$ à $C_6$, aryle, hétéroaryle, hétéroaryloxy, arylsulfone, alkylsulfone en $C_1$ à $C_7$, thioalkyle en $C_1$ à $C_7$ et aminoalkyle en $C_1$ à $C_7$ ; ou bien
  - un groupe alkynyle en $C_2$ à $C_7$ portant au moins un substituant choisi parmi l'oxygène, les halogènes et les groupes hydroxyle, formyle, carboxyle, aryloxycarbonyle, alkyloxycarbonyle en $C_2$ à $C_8$, alkényloxycarbonyle en $C_3$ à $C_9$, nitrile, aryle, hétéroaryle, arylsulfone, alkylsulfone en $C_1$ à $C_7$, thioalkyle en $C_1$ à $C_7$ et aminoalkyle $C_1$ à $C_7$ ; ou bien
  - un groupe alkényle ou alkynyle en $C_2$ à $C_{15}$ substitué par au moins un groupe trialkylsilyle en $C_1$ à $C_7$ ; tandis que

- $R_2$, qui peut être en position 3, 6 ou 8 sur le cycle quinoléique, représente un atome d'hydrogène ou d'halogène, un groupe hydroxyle, formyle, carboxyle, alkyle en $C_1$ à $C_7$, alcoxy en $C_1$ à $C_7$, amine, alkylamide en $C_1$-$C_{10}$, alkényle en $C_2$ à $C_7$ éventuellement substitué par un ou plusieurs groupes alcoxy en $C_1$ à $C_7$, ou encore un groupe alkynyle en $C_2$ à $C_{10,}$ ce dernier étant éventuellement substitué par un groupe hétéroaryle ;

ou l'un de ses sels pharmaceutiquement acceptables, à la condition toutefois que $R_1$ et $R_2$ ne soient pas tous deux un atome d'hydrogène, pour la préparation d'un médicament pour traiter les co-infections à protozoaires et à rétrovirus.

Conformément à l'Invention, dans la formule générale (I), les groupes alkyle, alcoxy, alkényle et alkynyle peuvent aussi bien être ramifiés que linéaires. Les termes *"aryle"* et *"aryl-"* désignent tout radical cyclique ou polycyclique ayant un caractère aromatique, tandis que les termes *"hétéroaryle"* et *"hétéroaryl-"* désignent tout radical cyclique ou polycyclique de nature aromatique et dont le ou les cycles comportent un ou plusieurs atomes choisi parmi l'azote, l'oxygène et le soufre. Par ailleurs, les quinoléines de formule générale (I) peuvent être utilisées sous leurs différentes formes stéréoisomères.

Parmi les quinoléines de formule générale (I), on préfère utiliser celles dont l'atome de carbone situé en position 2 du cycle quinoléique est substitué, c'est-à-dire celles dans lesquelles $R_1$ est différent d'un atome d'hydrogène.

De préférence, la ou les quinoléines sont choisies parmi les quinoléines dont l'atome de carbone situé en position 2 du cycle quinoléique est substitué par une chaîne hydrocarbonée insaturée, c'est-à-dire un groupe alkényle ou alkynyle porteur ou non de substituants, et, plus particulièrement, parmi celles dans lesquelles :

- $R_1$ représente :
  - un groupe alkényle ou alkynyle en $C_2$ à $C_{15}$ ; ou bien
  - un groupe alkényle en $C_2$ à $C_7$ portant au moins un substituant choisi parmi l'oxygène, les halogènes et les groupes hydroxyle, formyle, carboxyle, alkyloxycarbonyle en $C_2$ à $C_8$, nitrile, amine, alcoxy en $C_1$ à $C_7$, phénoxy, cycloalkyle en $C_3$ à $C_6$, aryle, hétéroaryle, hétéroaryloxy, arylsulfone, alkylsulfone en $C_1$ à $C_7$, thioalkyle en $C_1$ à $C_7$, aminoalkyle en $C_1$ à $C_7$ et trialkylsilyle, en particulier triméthylsilyle ; ou bien
  - un groupe alkynyle en $C_2$ à $C_7$ portant au moins un substituant choisi parmi l'oxygène, les halogènes et les groupes hydroxyle, formyle, carboxyle, alkyloxycarbonyle en $C_2$ à $C_8$, nitrile, aryle, hétéroaryle, arylsulfone, alkylsulfone en $C_1$ à $C_7$, thioalkyle en $C_1$ à $C_7$, aminoalkyle $C_1$ à $C_7$ et trialkylsilyle, en particulier triméthylsilyle ; tandis que

- $R_2$ représente un atome d'hydrogène, un groupe hydroxyle ou un groupe alkyle en $C_1$ à $C_7$. ,

De manière particulièrement préférée, la ou les quinoléines sont choisies parmi les quinoléines de formule générale (I) dans lesquelles $R_1$ représente un groupe alkényle ou alkynyle en $C_2$ à $C_7$ substitué par un plusieurs atomes d'halogène, notamment de chlore, de brome ou de fluor, les Inventeurs ayant en effet constaté que la présence de tels atomes se traduit généralement par des propriétés antiprotozoaires particulièrement prononcées.

Toutes les quinoléines de formule (I) peuvent être préparées par synthèse chimique, notamment selon les procédés décrits par Webb, Tetrahedron Lett., 1985, 26, 3191-3194, Fakhfakh et al, Tetrahedron Lett., 2001, 42, 3847-3850 et Fakhfakh et al., J. Organomet. Chem., 2001,624,131-135.

L'utilisation des quinoléines de formule générale (I) pour la préparation d'un médicament pour traiter les co-infections à protozoaires et à rétrovirus présente de nombreux avantages. En effet, elle permet, de par la double activité, antiprotozoaire et antirétrovirale, de ces quinoléines, de traiter les deux infections par une seule médication, ce qui favorise grandement l'observance et réduit considérablement les risques d'interactions médicamenteuses. Par ailleurs, bien que leur mécanisme d'action sur la réplication du VIH ne soit pas encore élucidé, les quinoléines de formule générale (I) apparaissent n'agir ni sur la transcriptase inverse, ni sur la protéase virale, en sorte que leur utilisation devrait permettre de s'affranchir des problèmes de résistance croisée auxquels se heurtent actuellement les cliniciens dans le traitement de l'infection par le HIV. En outre, ces quinoléines font preuve d'une absence de toxicité plaidant en faveur d'une tolérance très satisfaisante.

A titre d'exemples de co-infections susceptibles d'être traitées par un médicament préparé conformément à l'invention, on peut citer et sans que cela ait un quelconque caractère limitatif, les co-infections induites par un ou plusieurs protozoaires appartenant aux genres *Leishmania, Trypanosoma, Plasmodium, Toxoplasma, Pneumocystis* et *Schistosomia* et par un rétrovirus du type HIV ou HTLV-1.

De manière préférée, le médicament est destiné à traiter une co-infection *Leishmania*lVIH.

L'invention englobe, pour l'utilisation pour la préparation d'un médicament destiné à traiter les co-infections à protozoaires et à rétrovirus, à la fois des quinoléines qui ont déjà été décrites comme étant susceptibles de présenter une application thérapeutique et des quinoléines qui n'ont jamais été proposées en tant que médicaments.

L'invention a, donc, également pour objet une quinoléine répondant à la formule générale (I) représentée ci-avant dans laquelle :

- soit $R_1$ représente un groupe alkyle en $C_1$ à $C_7$, un groupe alkényle en $C_2$ à $C_7$, ou un groupe alkényle en $C_2$ à $C_7$ portant au moins un substituant choisi parmi les groupes aryle, auquel cas $R_2$, qui peut être en position 3, 6 ou 8 du cycle quinoléique, représente un groupe alkényle en $C_3$ à $C_7$ substitué par un ou plusieurs groupes alcoxy en $C_1$ à $C_7$, ou un groupe alkynyle en $C_2$ à $C_{10}$ substitué par un groupe hétéroaryle ;
- soit $R_1$ représente un groupe alkyle en $C_1$ à $C_7$ portant au moins un substituant choisi parmi les groupes hydroxyle, amine, alcoxy en $C_1$ à $C_4$ et aminoalkyle en $C_1$ à $C_{4,}$ ou $R_1$ représente un groupe alkényle en $C_2$ à $C_7$ portant au moins un substituant choisi parmi les groupes hydroxyle, amine, alcoxy en $C_1$ à $C_4$ et aminoalkyle en $C_1$ à $C_{4,}$ auquel cas $R_{2,}$ qui peut être en position 3, 6 ou 8 du cycle quinoléique, représente un groupe alkényle en $C_3$ à $C_7$ substitué par un ou plusieurs groupes alcoxy en $C_1$ à $C_{7,}$ ou un groupe alkynyle en $C_2$ à $C_{10}$, ce dernier étant éventuellement substitué par un groupe hétéroaryle ;
- soit $R_1$ représente :
  - un groupe méthyle ou éthyle portant au moins un substituant choisi parmi les halogènes ;

auquel cas $R_2$, qui peut être en position 3, 6 ou 8 du cycle quinoléique, représente un groupe alcoxy en $C_1$ à $C_7$, amine, alkylamide en $C_1$-$C_{10}$, alkényle en $C_2$ à $C_7$ substitué par un ou plusieurs groupes alcoxy en $C_1$ à $C_7$, ou encore un groupe alkynyle en $C_2$ à $C_{10}$, substitué par un groupe hétéroaryle ;

- soit $R_1$ représente :

un radical 2-pyridyle
auquel cas $R_2$, qui peut être en position 3, 6 ou 8 du cycle quinoléique, représente un atome d'hydrogène ou d'halogène, formyle, carboxyle, alkyle en $C_1$ à $C_7$, alcoxy en $C_1$ à $C_7$, amine, alkylamide en $C_1$-$C_{10}$, alkényle en $C_2$ à $C_7$ éventuellement substitué par un ou plusieurs groupes alcoxy en $C_1$ à $C_7$, ou encore un groupe alkynyle en $C_2$ à $C_{10}$, ce dernier étant éventuellement substitué par un groupe hétéroaryle;

- soit $R_1$ représente:

  • un groupe alkyle en $C_8$ à $C_{15}$, portant éventuellement un substituant choisi parmi les groupes aryle; ou bien
  • un groupe alkényle en $C_8$ à $C_{15}$, un groupe alkényle en $C_2$ à $C_7$ portant au moins un substituant choisi parmi les arylsulfone; ou bien
  • un groupe alkynyle en $C_2$ à $C_{15}$, un groupe alkynyle en $C_2$ à $C_7$ portant au moins un substituant choisi parmi les groupes aryle et arylsulfone ;

auquel cas $R_2$, qui peut être en position 3, 6 ou 8 du cycle quinoléique, représente un groupe choisi parmi un alcoxy en $C_1$ à $C_7$, une amine, un alkylamide en $C_1$-$C_{10}$, un alkényle en $C_2$ à $C_7$ éventuellement substitué par un ou plusieurs groupes alcoxy en $C_1$ à $C_7$, ou encore un groupe alkynyle en $C_2$ à $C_{10}$ substitué par un groupe hétéroaryle ;

- soit $R_1$ représente :

  • un atome d'hydrogène, un groupe formyle, un groupe hétéroaryle éventuellement substitué par un ou plusieurs groupes hydroxyle , à l'exception du radical 2-pyridyl ; ou bien
  • un groupe alkyle en $C_1$ à $C_{15}$ portant au moins un substituant choisi parmi l'oxygène et les groupes formyle, carboxyle, aryloxycarbonyle, alkyloxycarbonyle en $C_2$ à $C_8$, alkényloxycarbonyle en $C_3$ à $C_9$, nitrile, phénoxy, cycloalkyle en $C_3$ à $C_6$, hétéroaryloxy, arylsulfone, alkylsulfone en $C_1$ à $C_7$ et thioalkyle en $C_1$ à $C_7$, un groupe alkyle en $C_1$ à $C_7$ portant au moins un substituant choisi parmi les groupes alcoxy en $C_5$ à $C_7$ et aminoalkyle en $C_5$ à $C_7$, un groupe alkyle en $C_3$ à $C_{15}$ portant au moins un substituant choisi parmi les halogènes, un groupe alkyle en $C_6$ à $C_{15}$ substitué par au moins un groupe hétéroaryle, un groupe alkyle en $C_8$ à $C_{15}$ portant au moins un substituant choisi parmi les groupes hydroxyle, amine, alcoxy en $C_1$ à $C_7$ et aminoalkyle en $C_1$ à $C_7$ ; ou bien
  • un groupe alkényle en $C_2$ à $C_7$ portant au moins un substituant choisi parmi l'oxygène, les halogènes et les groupes carboxyle, aryloxycarbonyle, alkyloxycarbonyle en $C_2$ à $C_8$, alkényloxycarbonyle en $C_3$ à $C_9$, nitrile, phénoxy, cycloalkyle en $C_3$ à $C_6$, hétéroaryloxy, alkylsulfone en $C_1$ à $C_{7,}$ thioalkyle en $C_1$ à $C_{7,}$ alcoxy en $C_1$ à $C_7$ et aminoalkyle en $C_5$ à $C_{7\,;}$ un groupe alkényle en $C_3$ à $C_7$ substitué par un groupe hétéroaryle ; ou bien
  • un groupe alkynyle en $C_2$ à $C_7$ portant au moins un substituant choisi parmi l'oxygène, les halogènes et les groupes hydroxyle, formyle, carboxyle, aryloxycarbonyle, alkyloxycarbonyle en $C_2$ à $C_8$, alkényloxycarbonyle en $C_3$ à $C_9$, nitrile, hétéroaryle, alkylsulfone en $C_1$ à $C_7$, thioalkyle en $C_1$ à $C_7$ et aminoalkyle $C_1$ à $C_7$ ; ou bien encore
  • un groupe alkényle ou alkynyle en $C_2$ à $C_{15}$ substitué par au moins un groupe trialkylsilyle en $C_1$ à $C_7$ ;

auquel cas $R_2$, qui peut être en position 3, 6 ou 8 du cycle quinoléique, représente un atome d'hydrogène ou d'halogène, un groupe hydroxyle, formyle, carboxyle, alkyle en $C_1$ à $C_7$, alcoxy en $C_1$ à $C_7$, amine, alkylamide en $C_1$-$C_{10}$, alkényle en $C_2$ à $C_7$ éventuellement substitué par un ou plusieurs groupes alcoxy en $C_1$ à $C_7$, ou encore un groupe alkynyle en $C_2$ à $C_{10}$, ce dernier étant éventuellement substitué par un groupe hétéroaryle ; ou l'un de ses sels pharmaceutiquement acceptables à la condition toutefois que $R_1$ et $R_2$ ne soient pas tous deux un atome d'hydrogène, pour l'utilisation comme médicaments.

Là également, on préfère utiliser une quinoléine dont l'atome de carbone en position 2 du cycle quinoléique est substitué par une chaîne hydrocarbonée insaturée et, en particulier, une quinoléine dans laquelle :

- soit $R_1$ représente :

un groupe un groupe alkényle en $C_2$ à $C_7$ éventuellement substitué par un

ou plusieurs groupes choisis parmi les groupes hydroxyle, amine, aryle, alcoxy en $C_1$ à $C_4$ et aminoalkyle en $C_1$ à $C_4$,

auquel cas $R_2$ représente un groupe alkényle en $C_3$ à $C_7$ substitué par un ou plusieurs groupes alcoxy en $C_1$ à $C_7$, ou un groupe alkynyle en $C_2$ à $C_{10}$, ce dernier étant éventuellement substitué par un groupe hétéroaryle; .

- soit $R_1$ représente :
  - un groupe alkényle en $C_2$ à $C_7$ portant au moins un substituants choisi parmi l'oxygène, les halogènes et les groupes carboxyle, alkyloxycarbonyle en $C_2$ à $C_8$, nitrile, phénoxy, cycloalkyle en $C_3$ à $C_6$, hétéroaryloxy, alkylsulfone en $C_1$ à $C_7$, thioalkyle en $C_1$ à $C_7$, alcoxy en $C_5$ à $C_7$ et aminoalkyle en $C_5$ à $C_7$ ; un groupe alkényle en $C_3$ à $C_7$ substitué par un groupe hétéroaryle ; ou bien
  - un groupe alkynyle en $C_2$ à $C_7$ portant au moins un substituant choisi parmi l'oxygène, les halogènes et les groupes hydroxyle, formyle, carboxyle, alkyloxycarbonyle en $C_2$ à $C_8$, nitrile, hétéroaryle, alkylsulfone en $C_1$ à $C_7$, thioalkyle en $C_1$ à $C_7$ et aminoalkyle $C_1$ à $C_7$ ; ou bien encore
  - un groupe alkényle ou alkynyle en $C_2$ à $C_{15}$ substitué par au moins un groupe trialkylsilyle en $C_1$ à $C_7$ ;

  auquel cas $R_2$ représente un atome d'hydrogène, un groupe hydroxyle ou un groupe alkyle en $C_1$ à $C_7$.

De manière particulièrement préférée, $R_1$ représente un groupe alkényle ou alkynyle en $C_2$ à $C_7$ substitué par un plusieurs atomes d'halogène, en particulier de chlore, de brome ou de fluor.
L'invention englobe, pour l'utilisation comme médicaments, à la fois des quinoléines connues et des quinoléines nouvelles.
La présente invention a, en conséquence, aussi pour objet une nouvelle quinoléine qui est caractérisée en ce qu'elle répond à la formule générale (I) représentée ci-avant dans laquelle :

- soit $R_1$ représente un groupe cyclopropyl-hydroxyméthyle, 4-chloro-but-3-en-1-yn-1-yle, hept-1-en-1-yle, 2-bromo-éthényle, 2-bromoéthynyle, 2-bromo-2-fluoro-éthényle, 4-méthylcarboxylate-but-1,3-dien-1-yle, dec-1-yn-1-yle, 2-(2-quinoléyl)-éthényle, 2-(triméthylsilyl-éthynyl)-4-triméthylsilyl-but-1-en-3-yn-1-yle, auquel cas $R_2$ représente un atome d'hydrogène ;
- soit $R_1$ représente un groupe prop-l-en-l-yle, auquel cas $R_2$ représente un groupe méthyle en position 6 ou un groupe hydroxyle en position 8 du cycle quinoléique ;
- soit $R_1$ représente un groupe 2-hydroxypropyle, auquel cas $R_2$ représente un groupe hydroxyle en position 8 du cycle quinoléique ;
- soit encore $R_1$ représente un groupe 2-méthylcarboxylate-éthényle, auquel cas $R_2$ représente un groupe méthyle en position 6 du cycle quinoléique.

La présente invention a, en outre pour objet, une composition pharmaceutique comprenant, à titre de principe actif, au moins une nouvelle quinoléine telle que définie ci-avant.
D'autres avantages et caractéristiques de l'invention apparaîtront à la lecture du complément de description qui suit et qui se réfère à des exemples de préparation de quinoléines substituées entrant dans le cadre de l'invention et de démonstration de leur activité biologique *in vitro.*
Il va de soi, toutefois, que ces exemples sont donnés uniquement à titre d'illustrations de l'invention et n'en constituent nullement une limitation.

## I - PREPARATION DE QUINOLEINES SUBSTITUEES

**Exemple 1 : Préparation du 2-(2-quinolyl)-triméthylsilylacétylène** [quinoléine de formule générale (I) dans laquelle $R_1 = -C\equiv C-Si(CH_3)_3$ et $R_2$ = H]

Dans un ballon sec muni d'un barreau aimanté et d'une entrée d'azote, on dissout 0,6 g (4,13 mmol) de N-oxyde de quinoléine dans 15 ml de tétrahydrofurane (THF) anhydre à 20°C. La solution résultante est traitée par 0,64 ml (4,96 mmol) de chloroformate d'isobutyle et la suspension obtenue est refroidie à -78°C.
6,23 ml d'une solution de bromure de triméthylsilyléthynyl magnésium dans du THF (préparée à partir d'un mélange de 1,28 ml (9 mmol) de triméthylsilylacétylène et de 4,95 ml (9,9 mmol) d'une solution 2M de bromure de butylmagnésium dans du THF) sont ensuite ajoutés goutte à goutte. Après 90 minutes d'agitation à -78°C, la solution est lentement ramenée à température ambiante (30 minutes), puis hydrolysée par 15 ml d'eau.
Après séparation des deux phases, la phase aqueuse est extraite avec de l'éther diéthylique (5 x 50 ml). Les phases organiques sont rassemblées, le solvant est évaporé et le résidu est repris par 10 ml d'acide sulfurique (2 M). La solution acide est extraite avec du dichlorométhane (2 x 75 ml). Puis, la phase aqueuse est ramenée à pH = 9 à l'aide d'une solution saturée de $K_2CO_3$, et extraite avec du dichlomméthane (2 x 50 ml).

Les phases organiques issues de l'extraction acide sont rassemblées, lavées avec une solution saturée de $K_2CO_3$, puis une solution saturée de NaCl, séchées sur sulfate de magnésium ($MgSO_4$), filtrées et concentrées.
Les phases organiques issues de l'extraction basique sont lavées avec une solution saturée de NaCl, séchées sur sulfate de magnésium ($MgS0_4$), ,filtrées et concentrées.
Les deux bruts réactionnels sont rassemblés et purifiés par chromatographie moyenne pression sur gel de silice en utilisant comme éluant : cyclohexane/acétate d'éthyle : 95/5, pour obtenir 0,696 g de 2-(2-quinolyl)-triméthylsilylacétylène.

Rendement : 75%.
Formule Brute: $C_{14}H_{15}NSi$.
$^1H$ RMN (200 MHz, $CDCl_3$)-$\delta$ ppm : 0,30 (s, 9H); 7,52 (m, 2H); 7,72 (m, 2H); 8,08 (d, J = 8,4 Hz, 1H); 8,1 (d, J = 8,3 Hz, 1H).
$^{13}C$ RMN (50 MHz, $CDCl_3$)-$\delta$ ppm : -0,35; 95,50; 104,19; 124,23; 127,02; 127,50; 127,31; 129,26; 129,84 ; 135,90; 143,09; 147,94.
MS-IC ($NH_4^+$): 225 ($M^+$,100).
IR ($cm^{-1}$): 2955; 2155; 1590; 1555; 1500; 1455; 1420; 1375; 1330; 1305; 1245; 1220; 1115; 955; 905; 840; 820; 790; 765; 745; 700; 635.

**Exemple 2 : Préparation du 1-(2-quinolyl)-acétylène** [quinoléine de formule générale (I) dans laquelle $R_1$ = -C≡CH et $R_2$ = H]

Dans un ballon sec muni d'un barreau aimanté et d'une entrée d'azote, on dissout 0,075 g (0,33 mmol) de 2-(2-quinolyl)-triméthylsilylacétylène obtenu comme décrit dans l'exemple 1, dans 8 ml de THF anhydre. A cette solution, on ajoute 0,36 ml (0,36 mmol) de fluorure de n-tétrabutylammonium d'une solution 1M dans le tétrahydrofurane. Après 15 minutes d'agitation à température ambiante, la réaction est hydrolysée par 10 ml d'eau.
Après séparation des deux phases, la phase aqueuse est extraite avec de l'acétate d'éthyle (4 x 50 ml).
Les phases organiques sont rassemblées, lavées avec une solution saturée d'hydrogénocarbonate de sodium ($NaHCO_3$), puis une solution saturée de NaCl, séchées sur sulfate de magnésium ($MgSO_4$), filtrées et concentrées.
Le brut réactionnel obtenu est purifié par chromatographie moyenne pression sur gel de silice en utilisant comme éluant : cyclohexane/acétate d'éthyle : 80/20, pour obtenir 0,045 g de 1-(2-quinolyl)-acétylène.

Rendement : 88%.
Formule Brute : $C_{11}H_7N$.
$^1H$ RMN (400 MHz, $CDCl_3$)-$\delta$ ppm : 3,25 (s, 1H, H-2'); 7,55 (d, J = 8,7 Hz, 1H, H-3); 7,56 (t, J = 7,7 Hz, 1H, H-6); 7,74 (dd, J = 7,6, 7,6 Hz, 1H, H-7); 7,81 (d, J = 8,1 Hz, 1 H, H-5); 8,11 (d, J = 8,8 Hz, 1 H, H-8); 8,4 (d, J = 8,7 Hz, 1 H, H-4).
$^{13}C$ RMN (50 MHz, $CDCl_3$)-$\delta$ ppm : 77,48 (C-2'); 83,11 (C-1'); 123,80 (C-3), 127,04 (C-6); 127,18 (C-5); 127,40 (C-10); 128,97 (C-8); 129,78 (C-7); 135,91 (C-4); 142,06 (C-2); 147,68 (C-9).
MS-ESI : 154 ($MH^+$, 100).
IR ($cm^{-1}$) : 3165; 2105; 1615; 1595; 1555; 1500; 1420; 1375; 1330; 1305; 1255; 1210; 1140; 1115; 955; 830; 620.

**Exemple 3 : Préparation du *E*-3-(2-quinolyl)-2-propénoate de méthyle** [quinoléine de formule générale (I) dans laquelle $R_1$ = -CH=CH-C(O)$OCH_3$ et $R_2$ = H]

Dans un ballon sec muni d'un barreau aimanté et d'une entrée d'azote, on dissout 0,2 g (1,27 mmol) de 2-formylquinoléine dans 10 ml de toluène anhydre. On ajoute 0,51 g (1,52 mmol) de méthyl(triphénylphosphoranylidène) acétate. Le mélange réactionnel est porté aux reflux du toluène pendant 90 minutes, puis la solution est lentement ramenée à température ambiante et hydrolysée par 10 ml d'eau.
Après séparation des deux phases, la phase aqueuse est extraite avec de l'acétate d'éthyle (4 x 50 ml). Les phases organiques rassemblées sont lavées avec une solution saturée d'hydrogénocarbonate de sodium ($NaHCO_3$), puis une solution saturée de NaCl, séchées sur sulfate de magnésium ($MgSO_4$), filtrées et concentrées.
Le brut réactionnel obtenu est purifié par chromatographie moyenne pression sur gel de silice en utilisant comme éluant : cyclohexane/acétate d'éthyle : 70/30, pour obtenir 0,216 g de *E*-3-(2-quinolyl)-2-propénoate de méthyle.

Rendement : 80%.
Formule Brute : $C_{13}H_{11}NO_2$.
$^1H$ RMN (200 MHz, $CDCl_3$)-$\delta$ ppm : 3,85 (s, 3H, OMe); 7,00 (d, J = 14,9 Hz, 1H, H-2'); 7,54 (t, J = 7,9 Hz, 1H, H-6); 7,57 (d, J = 8,7 Hz, 1H, H-3); 7,72 (dd, J = 7,2, 8,1 Hz, 1H, H-7); 7,78 (d, J = 8,1 Hz, 1H, H-5); 7,89 (d, J = 16,0 Hz, 1H, H-1'); 8,10 (d, J = 8,5 Hz, H-8); 8,14 (d, J = 8,5 Hz, H-4).

13$_C$ RMN (50 MHz, $CDCl_3$)-δ ppm : 51,67 (OMe); 120,12 (C-3); 123,01 (C-2’), 127,11 (C-6); 127,33 (C-5); 127,87 (C-10); 129,67 (C-8); 129,83 (C-7); 136,48 (C-4); 144,11 (C-1’ 148,06 (C-9); 152,88 (C-2); 166,74 (C-3’).
MS-ESI : 214 ($MH^+$, 100).
IR ($cm^{-1}$): 1740 (C=O); 1725; 1650; 1595; 1560; 1500; 1435; 1345; 1245; 1195; 1155; 975; 825; 755; 715; 620.

**Exemple 4 : Préparation du *E*-3-(2-quinolyl)-pron-2-en-1-ol** [quinoléine de formule générale (I) dans laquelle $R_1$ = $-CH=CH-CH_2OH$ et $R_2$ = H]

Dans un ballon sec muni d’un barreau aimanté et d’une entrée d’azote, on dissout 0,2 g (0,93 mmol) de E-3-(2-quinolyl)-2-propénoate de méthyle obtenu comme décrit dans l’exemple 3, dans 10 ml de toluène anhydre. La solution est refroidie à -78°C puis est ajouté goutte à goutte 1,87 ml (1,87 mmol) d’hydrure de di-isobutylaluminium en solution 1M dans le toluène. Après 2 heures d’agitation, 5 ml de méthanol sont additionnés, puis la solution est lentement ramenée à température ambiante et 50 ml d’acétate d’éthyle sont ajoutés, l’hydrolyse est poursuivie toute la nuit puis la solution est filtrée sur célite. Le filtrat est extrait avec de l’acétate d’éthyle (2 x 25 ml).
Les phases organiques sont rassemblées, lavées avec une solution saturée d’hydrogénocarbonate de sodium ($NaHCO_3$), puis une solution saturée de NaCl, séchées sur sulfate de magnésium ($MgSO_4$), filtrées et concentrées.
Le brut réactionnel obtenu est purifié par chromatographie moyenne pression sur gel de silice en utilisant comme éluant : cyclohexane/acétate d’éthyle : 60/40, pour obtenir 0,085 g de *E*-3-(2-quinolyl)-prop-2-en-1-ol.

Rendement : 49%.
Formule Brute : $C_{12}H_{11}NO$.
$^1H$ RMN (200 MHz, $CDCl_3$)-δ ppm : 4,44 (d, J = 3,9 Hz, 2H, H-3’); 6,87 (dt, J = 16,8, 4,3 Hz, 1H, H-2’); 7,01 (d, J = 16,3 Hz, 1H, H-1’); 7,44 (m, 2H); 7,67 (m, 2H); 7,57 (m, 2H).
13$_C$ RMN (50 MHz, $CDCl_3$)-δ ppm : 62,60 (C-3’); 118,90; 126,15; 127,21; 127,40; 128,73; 129,73; 130,11; 136,48; 137,21; 147,67; 155,71.
MS-ESI : 208 (M+Na, 12); 186 ($MH^+$, 100).
IR ($cm^{-1}$) : 3145; 2835; 1650; 1615; 1600; 1560, 1505; 1430; 1370; 1315; 1150; 1120; 1095; 965; 930; 840; 810; 770; 745; 635; 605.

**Exemple 5 : Préparation du 3-(2-quinolyl)-propénal** [quinoléine de formule générale (I) dans laquelle $R_1$ = $-CH=CH-COH$ et $R_2$ = H]

Dans un ballon sec muni d’un barreau aimanté et d’une entrée d’azote, on dissout 0,2 g (1,27 mmol) de 2-formylquinoléine dans 10 ml de toluène anhydre. A cette solution, on ajoute 0,46 g (1,52 mmol) de (triphénylphosphoranylidène)-acétaldéhyde. Le mélange est porté aux reflux du toluène pendant 90 minutes, puis la solution est lentement ramenée à température ambiante et hydrolysée par 10 ml d’eau. Après séparation des deux phases, la phase aqueuse est extraite avec de l’acétate d’éthyle (4 x 50 ml).
Les phases organiques sont rassemblées, lavées avec une solution saturée d’hydrogénocarbonate de sodium ($NaHCO_3$), puis une solution saturée de NaCl, séchées sur sulfate de magnésium ($MgSO_4$), filtrées et concentrées.
Le brut réactionnel obtenu est purifié par chromatographie moyenne pression sur gel de silice en utilisant comme éluant : cyclohexane/acétate d’éthyle : 75/25, pour obtenir 0,137 g de 3-(2-quinolyl)-propénal.

Rendement : 60%.
Formule Brute : $C_{12}H_9NO$.
$^1H$ RMN (400 MHz, $CDCl_3$)-δ ppm : 7,13 (ddd, J = 16,2, 7,7, 0,7 Hz, 1H, H-2’); 7,59 (dd, J = 7,0, 7,0 Hz, 1H, H-6); 7,69 (d, J = 8,5 Hz, 1H, H-3); 7,73 (d, J = 16,1 Hz, 1H, H-1’); 7,76 (dd, J = 7,2, 7,5 Hz, 1H, H-7); 7,84 (d, J = 8,1 Hz, 1H, H-5); 8,12 (d, J = 8,5 Hz, 1H, H-8); 8,22 (d, J = 8,5 Hz, 1H, H-4); 9,86 (dd, J = 7,7, 0,7 Hz, 1H, H-3’).
$^{13}C$ RMN (50 MHz, $CDCl_3$)-δ ppm : 119,85 (C-3); 127,52 (C-5), 127,78 (C-6); 128,11 (C-10); 129,91 (C-8); 130,22 (C-7); 132,54 (C-2’); 136,84 (C-4); 148,28 (C-9); 151,75 (C-1’); .152,84 (C-2); 193,52 (C-3’).
MS-ESI : 184 ($MH^+$, 100).
IR ($cm^{-1}$): 3050; 1670 (C=O); 1630; 1595; 1555; 1500; 1430; 1290; 1125; 1110; 980; 900; 825; 785; 625; 590.

**Exemple 6 : Préparation du (2-quinolyl)-éthylène** [quinoléine de formule générale (I) dans laquelle $R_1$ = $-CH=CH_2$ et $R_2$ = H]

Dans un ballon sec muni d’un barreau aimanté et d’une entrée d’azote, on dissout 0,8 g (5,51 mmol) de N-oxyde de quinoléine dans 15 ml de THF anhydre à 20°C. Cette solution est traitée par 0,78 ml (6,07 mmol) de chloroformate d’isobutyle ; la suspension obtenue est refroidie à -78°C. Puis, 11 ml (11 mmol) d’une solution 1 M dans du THF de

bromure de vinylmagnésium sont ajoutés goutte à goutte. Après 90 minutes d'agitation à -78°C, la solution est lentement ramenée à température ambiante (30 minutes), puis hydrolysée par 15 ml d'eau.

Après séparation des deux phases, la phase aqueuse est extraite avec de l'éther diéthylique (5 x 50 ml). Les phases organiques sont rassemblées, le solvant est évaporé et le résidu est repris par 10 ml d'acide sulfurique (2M). La solution acide est extraite avec du dichlorométhane (2 x 75 ml) et la phase aqueuse est ramenée à pH = 9 à l'aide d'une solution saturée de $K_2CO_3$, puis extraite avec $CH_2Cl_2$ (2 x 50 ml).

Les phases organiques issues de l'extraction acide sont rassemblées, lavées avec une solution saturée de $K_2CO_3$, puis une solution saturée de NaCl, séchées sur sulfate de magnésium ($MgSO_4$), filtrées et concentrées.

Les phases organiques issues de l'extraction basique sont lavées avec une solution saturée de NaCl, séchées sur sulfate de magnésium ($MgSO_4$), filtrées et concentrées.

Les deux bruts réactionnels sont rassemblés et purifiés par chromatographie moyenne pression sur gel de silice en utilisant comme éluant : cyclohexane/acétate d'éthyle : 95/ 5, pour obtenir 0,126 g de (2-quinolyl)-éthylène.

Rendement : 82%.

Formule Brute : $C_{11}H_9N$.

$^1H$ RMN (200 MHz, $CDCl_3$)-δ ppm : 5,66 (d, J = 10,9 Hz, 1H, H-2'a); 6,27 (d, J =17,7 Hz, 1H, H-2'b); 7,04 (dd, J = 17,7, 11,1 Hz, 1H, H-1'); 7,49 (t, J = 7,2 Hz, 1 H, H-6); 7,60 (d, J = 8,7 Hz, 1 H, H-3); 7,69 (dd, J = 7,2, 8,1 Hz, 1 H, H-7); 7,77 (d, J = 7,9 Hz, 1H, H-5); 8,06 (d, J = 9,8 Hz, 1H, H-8); 8,11 (d, J = 8,0 Hz, 1H, H-4).

$^{13}C$ RMN (50 MHz, $CDCl_3$)-δ ppm : 118,24 (C-3); 119,66 (C-2'), 126,17 (C-6); 127,11 (C-10); 127,32 (C-5); 129,22 (C-8); 129,46 (C-7); 136,17 (C-4); 137,87 (C-1'); 147,93 (C-9); 155,96 (C-2).

IR ($cm^{-1}$) :1615; 1595; 1555; 1505; 1425; 1310; 1140; 1120; 1015; 990; 925; 830; 760; 715; 730; 615.

**Exemple 7 : Préparation du *E*-1-(2-quinol)-butène** [quinoléine de formule générale (I) dans laquelle $R_1$ = -CH=CH-$CH_2$-$CH_3$ et $R_2$ = H]

Dans un ballon sec muni d'un barreau aimanté et d'une entrée d'azote, 4,90 g (12,7 mmol) de bromure de propyltriphénylphosphonium sont mis en suspension dans 15 ml de toluène anhydre à 0°C, puis 1,71 g (15,2 mmol) de *tert*-butanolate de potassium (tBuOK) sont ajoutés. Après 15 minutes d'agitation, une solution à 0°C contenant 1 g (6,36 mol) de 2-formylquinoléine dans 10 ml de tétrahydrofurane anhydre est ajoutée. Le mélange réactionnel est porté aux reflux du toluène pendant 90 minutes, puis la solution est lentement ramenée à température ambiante et hydrolysée par 20 ml d'eau.

Après séparation des deux phases, la phase aqueuse est extraite avec l'acétate d'éthyle (4 x 50 ml).

Les phases organiques sont rassemblées, lavées avec une solution saturée d'hydrogénocarbonate de sodium ($NaHCO_3$), puis une solution saturée de NaCl, séchées sur sulfate de magnésium ($MgSO_4$), filtrées et concentrées.

Le brut réactionnel obtenu est purifié par chromatographie moyenne pression sur gel de silice en utilisant comme éluant : cyclohexane/acétate d'éthyle : 92/8, pour obtenir 0,674 g de E-1-(2-quinolyl)-butène.

Rendement : 58%.

Formule Brute : $C_{13}H_{13}N$.

$^1H$ RMN (400 MHz, $CDCl_3$)-δ ppm : 1,17 (t, J = 7,5 Hz, 3H, H-4'); 2,36 (dqd, J = 7,5, 6,3, 1,5 Hz, 1H, H-3'); 6,72 (dt, J = 15,9, 1,5 Hz, 1H, H-1'); 6,88 (dd, J = 15,9, 6,3 Hz, 1H, H-2'); 7,45 (ddd, J = 6,9, 6,9, 1,2 Hz, 1H, H-6); 7,52 (d, J = 8,6 Hz, 1H, H-3); 7,66 (ddd, J = 6,9 , 6,9, 1,5 Hz, 1H, H-7); 7,74 (dd, J = 8,01, 1,3 Hz, 1H, H-5); 8,03 (dd, J = 9,4 Hz, 1H, H-8); 8,06 (d, J = 9,0 Hz, 1H, H-4).

$^{13}C$ RMN (50 MHz, $CDCl_3$)-δ ppm : 12,90 (C-4'); 25,80 (C-3'); 118,46 (C-3); 125,54 (C-6); 126,90 (C-10); 127,18 (C-5); 128,88 (C-8); 129,21 (C-7); 129,92 (C-1'); 135,84 (C-4); 139,00 (C-2'); 147,87 (C-9); 156,30 (C-2).

MS-ESI : 184 ($MH^+$, 100).

IR($cm^{-1}$): 2965; 1650; 1615; 1595; 1555; 1505; 1460; 1425; 1315; 1115; 965; 855; 815; 750; 620.

**Exemple 8 : Préparation du E-1-bromo-2-(2-quinolyl)-éthène** [quinoléine de formule générale (I) dans laquelle $R_1$ = -CH=CH-Br et $R_2$ = H]

Dans un ballon sec muni d'un barreau aimanté et d'une entrée d'azote, on dissout 0,2 g ($6,38.10^{-4}$ mol) de 1,1-dibromo-2-(2-quinolyl)éthène obtenu à partir de 2-formylquinoléine, de tétrabromocarbone ($CBr_4$), de triphénylphosphine ($PPh_3$) (Ramirez et *al., J. Am. Chem. Soc.,* 1962, 84 : 1745) et 11,28 mg ($3,19.10^{-5}$ mol, 5mol %) de triacétylacétonate de fer ($Fe(acac)_3$) dans 3 ml de THF anhydre et 3 ml de N-méthylpyrrolydinone (NMP) anhydre à -10°C. A cette solution, on ajoute goutte à goutte 0,39 ml ($7,02.10^{-4}$ mol) d'une solution 1,8M de bromure d'isopropylmagnésium dans du THF. Après 30 minutes d'agitation, la réaction est hydrolysée par 10 ml d'eau et la solution est ramenée à température ambiante. Ensuite, les deux phases sont séparées et la phase aqueuse est extraite avec de l'éther diéthylique

(3 x 30 ml).

Les phases organiques sont rassemblées, lavées avec une solution saturée d'hydrogénocarbonate de sodium ($NaHCO_3$) puis une solution saturée de NaCl, séchées sur sulfate de magnésium ($MgSO_4$) filtrées et concentrées.

Le brut réactionnel obtenu est purifié par chromatographie moyenne pression sur gel de silice en utilisant comme éluant : hexane/acétate d'éthyle : 92/8, pour obtenir 0,125 g de *E*-1-bromo-2-(2-quinolyl)-éthène.

Rendement : 84%.
Formule Brute : $C_{11}H_8NBr$
$^1H$ RMN (400 MHz, $CDCl_3$)-$\delta$ ppm: 7,36 (d, J = 8,37 Hz, 1H); 7,37 (d, J = 15,9 Hz, 1H); 7,50 (d, J = 13,7 Hz, 1H); 7,52 (t, J = 3,55 Hz, 1H); 7,70 (t, J = 7,5 Hz, 1 H); 7,76 (d, J = 8,11 Hz, 1H); 8,04 (d, J = 8,5 Hz, 1 H); 8,10 (d, J = 8,4 Hz, 1 H).
$^{13}C$ RMN (50 MHz, $CDCl_3$)-$\delta$ ppm: 114,14 (C-2'); 119,24 (C-3); 126,51(C-6); 126,75 (C-5) ; 127,51(C-10); 129,35 (C-8); 129,97 (C-7); 136,71 (C-4); 137,53 (C-1'); 147,06 (C-9); 154 (C-2).
MS-ESI : 236 ($MH^+$, 100); 234 ($MH^+$, 78).
IR ($cm^{-1}$) : 3055; 1605; 1590; 1550; 1500; 1425; 1305; 1140; 1115; 935; 835; 800; 775; 745; 620; 575.

## II - ACTIVITE BIOLOGIQUE *IN VITRO* DES QUINOLEINES SUBSTITUEES

L'activité biologique antiprotozoaire des quinoléines substituées a été mise en évidence sur :

- des amastigotes de *Leishmania amazonensis* (référence OMS : MPRO/BR/1972/M1841), de *Leishmania donovani* (MHOM/ET/67/L82) et de *Leishmania infantum* (MHOM/MA(BE)67 et IPZ229/1/89) ;
- des amastigotes de *Trypanosoma cruzi* (souche Tulahuen) et des formes circulantes de *Trypanosoma brucei* (souche S427).

Leur activité antirétrovirale a été, elle, mise en évidence sur des cellules CEM4fx infectées par le clone moléculaire pLN4-3 du virus de l'immunodéficience humaine (VIH-1).

### 1) Quinoléines substituées testées :

Les quinoléines substituées dont l'activité biologique a été testée sont représentées dans le Tableau I ci-après.

TABLEAU I

| Numéro | Poids moléculaire | $R_1$ | $R_2$ |
|---|---|---|---|
| 1 | 153 | 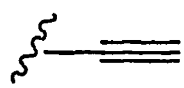 | H |
| 2 | 155 | 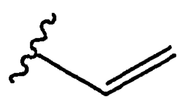 | H |
| 4 | 169 | H | 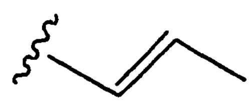 |
| 5 | 171 | 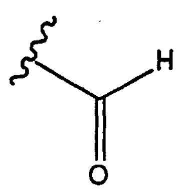 | $CH_3$ en position 6 |
| 6 | 183 | 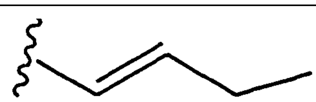 | H |

(suite)

| Numéro | Poids moléculaire | $R_1$ | $R_2$ |
|---|---|---|---|
| 7 | 183 | 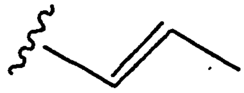 | $CH_3$ en position. 6 |
| 8 | 183 | 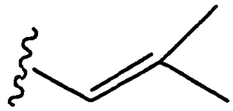 | H |
| 9 | 183 | 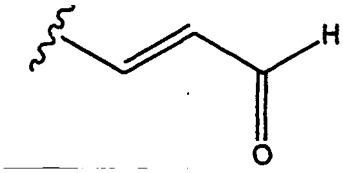 | H |
| 10 | 185 | 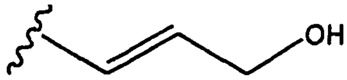 | H |
| 11 | 185 | 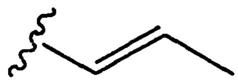 | OH en position 8 |
| 13 | 185 | 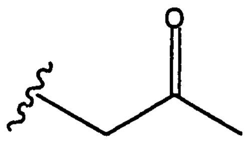 | H |
| 14 | 185 | H | 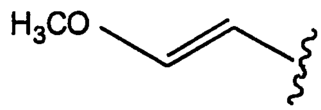 en position 6 . |
| 15 | 187 | 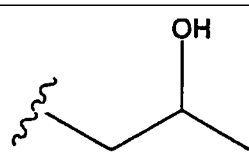 | H |
| 16 | 187 | 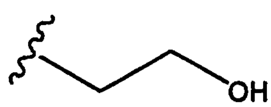 | H |
| 17 | 187 | 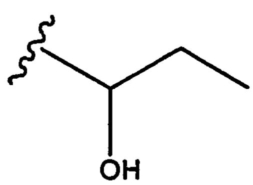 | H |
| 18 | 197 | 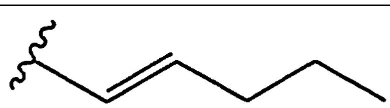 | H |

(suite)

| Numéro | Poids moléculaire | $R_1$ | $R_2$ |
|---|---|---|---|
| 19 | 199 | 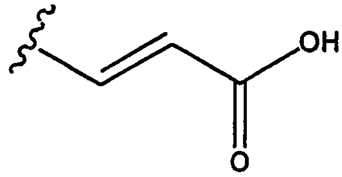 | H |
| 20 | 199 | 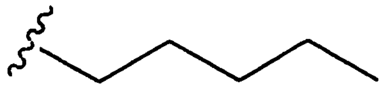 | H |
| 21 | 201 | 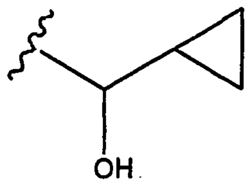 | H |
| 22 | 203 | 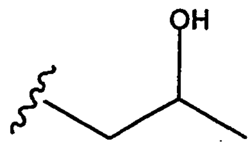 | OH en position 8 |
| 23 | 213 | 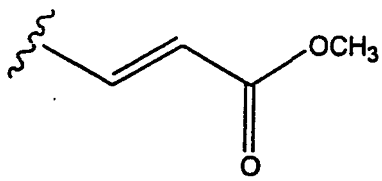 | H |
| 24 | 213 | 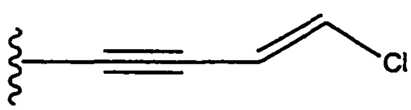 | H |
| 25 | 225 | 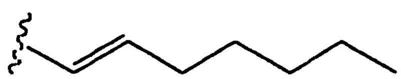 | H |
| 26 | 225 | 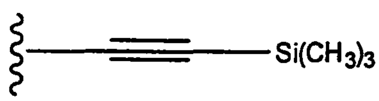 | H |
| 27 | 227 | 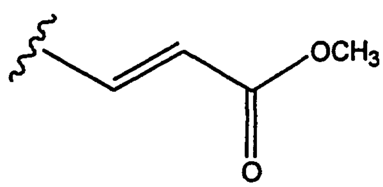 | $CH_3$ en position 6 |
| 28 | 231,9 | 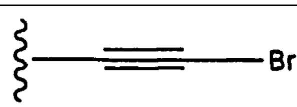 | H |

(suite)

| Numéro | Poids moléculaire | $R_1$ | $R_2$ |
|---|---|---|---|
| 29 | 233,9 | 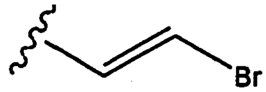 | H |
| 30 | 239 | 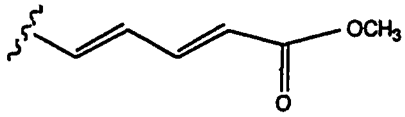 | H |
| 31 | 251,9 | 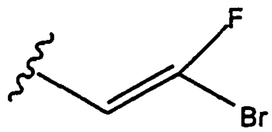 | H |
| 32 | 265 | 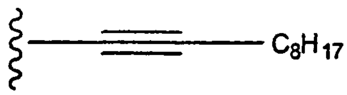 | H |
| 33 | 265 | H | 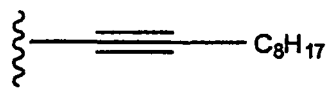 en position 3 |
| 34 | 277 | 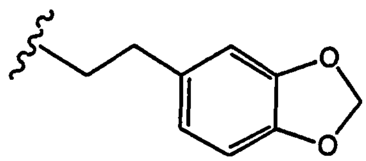 | H |
| 35 | 282 | 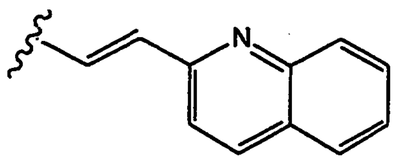 | H |
| 36 | 313 | 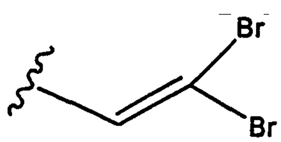 | H |
| 37 | 332 | H | 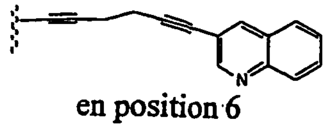 en position 6 |

(suite)

| Numéro | Pois moléculaire | $R_1$ | $R_2$ |
|---|---|---|---|
| 38 | 345 | 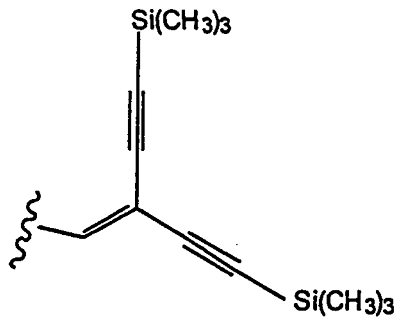 | H |

2) **Protocoles expérimentaux :**

a) **Essais effectués sur les amastigotes de *Leishmania amazonensis* :**

La souche de *Leishmania amazonensis* est maintenue par passages successifs dans des souris *"nude"* (nu/nu). Les amastigotes sont isolés à partir des lésions développées au niveau des coussinets plantaires et purifiés selon la méthode décrite par Antoine et al. (Parasitology, 1989, 99 : 1).

Les macrophages, qui vont servir de cellules hôtes pour les leishmanies, sont obtenus après multiplication et différenciation de précurseurs médullaires de souris BALB/c. Cette culture est réalisée en présence de surnageant d'une lignée conditionnée de fibroblastes L-929 source de *"Macrophage Colony Stimulating Factor"*. Après 5 jours de culture sur support hydrophobe, les macrophages adhérents sont récupérés puis distribués dans des plaques de 96 puits à fond plat pour culture cellulaire à raison de $4.10^4$ macrophages par puits. Les macrophages sont ensuite infectés avec des amastigotes purifiés (4 parasites par macrophage) et incubés à 34 °C pendant 24 heures pour permettre le développement des vacuoles parasitophores et la multiplication des parasites. Dans ces conditions, plus de 95 % des macrophages sont infectés.

Les solutions des quinoléines substituées à tester sont préparées en DMSO avant d'être ajoutées, à différentes concentrations, aux cultures de macrophages infectés. Des dilutions sérielles en base 2 à partir de 100 μg/ml jusqu'à # 6 ng/ml ont été utilisées et la concentration finale en DMSO, qui est dans tous les cas de 0,1%, s'est montrée non toxique vis-à-vis des macrophages.

La détermination de l'activité leishmanicide des quinoléines s'effectue 30 heures après l'addition des solutions de quinoléines substituées. Elle est basée sur la diminution plus ou moins forte, voire complète, d'une part, de la taille des vacuoles parasitophores et, d'autre part, du nombre d'amastigotes intracellulaires vivants. On détermine ainsi, pour chaque quinoléine substituée testée, la concentration inhibant 100% des parasites (ICioo).

b) **Essais sur les amastigotes de *Leishmania donovani* et *Leishmania infantum***

Les souches *Leishmania donovani* et *Leishmania infantum* sont maintenues par passages successifs dans des hamsters dorés. Les amastigotes sont isolés à partir des rates des hamsters infectés, et mis en contact avec les macrophages purifiés.

Vingt-quatre heures après l'injection intrapéritonéale d'une solution d'amidon à 2% à des souris Balb/c, les macrophages péritonéaux de ces souris sont collectés, lavés en PBS Dulbecco puis distribués dans des chambres de culture Labtek® à raison de $4.10^4$ cellules par 100 μl par puits en milieu RPMI-1640 supplémenté avec 10 % de SVF et des antibiotiques. Après 24 heures à 37°C en atmosphère humide (5% $CO_2$), les macrophages sont mis en contact avec des amastigotes purifiés (3 parasites par macrophage) dans un volume final de 200 μl pendant 4 heures. Le milieu est ensuite retiré pour éliminer les parasites extracellulaires et remplacé par 100 μl de milieu frais.

Vingt-quatre heures après le début de l'infection, les solutions de quinoléines substituées préparées comme décrit dans le paragraphe a) ci-avant, sont ajoutées et l'activité leishmanicide est déterminée au bout de 48 heures par comptage du nombre de parasites encore vivants dans les cellules fixées et colorées au Giemsa On détermine ainsi, pour chaque quinoléine substituée testée, la concentration inhibant 50 % des parasites ($CI_{50}$).

### c) Essais sur les amastigotes de *Trypanosoma cruzi*

Des macrophages péritonéaux de souris Balb/c sont collectés, lavés en PBS-Dulbecco puis distribués dans des plaques 96 puits à raison de $3.10^4$ cellules par 100 µl et par puits en milieu RPMI-1640 supplémenté avec 10 % SVF et des antibiotiques.

Après 24 heures à 37°C, $10^5$ trypomastigotes sont ajoutés avec des dilutions sérielles en base 2 des différentes quinoléines substituées. Les cultures sont ensuite incubées à 37°C pendant 4 jours en atmosphère humide (5% $CO_2$-air 95%). Après fixation, puis coloration au Giemsa, l'activité antiparasitaire est appréciée par comptage des trypomastigotes extracellulaires et des amastigotes intracellulaires. On détermine ainsi, pour chaque quinoléine substituée testée, la concentration inhibant 50% des parasites ($CI_{50}$).

### d) Essais sur les formes circulantes de *Trypanosoma brucei*

Les formes circulantes de *Trypanosoma brucei* sont cultivées en milieu MEM (milieu essentiel minimum; GIBCO-BRL, n° 072-1100) additionné de sels de Earle et supplémenté avec 1 mg/ml de glucose, 2,2 mg/ml de $NaHCO_3$, 10 mM d'HEPES, 2 mM de pyruvate de sodium, 0,2 mM de 2-mercaptoéthanol, 0,1 mM d'hypoxanthine et 15 % de sérum de cheval inactivé.

Les cultures sont maintenues à 37°C en plaques 24 puits en atmosphère humide (5% $CO_2$-95% air) et les passages effectués tous les 2 ou 3 jours à une densité de $10^3$ à $10^5$ trypomastigotes par ml. L'activité des quinoléines substituées est testée en triplicate dans des plaques 96 puits dans lesquelles $10^3$ parasites provenant d'une phase exponentielle de croissance sont distribués par puits, en présence des différentes solutions de quinoléines substituées dans un volume final de 200 µl. Après 48 heures de culture, l'activité trypanocide est évaluée par comptage à la cellule de Neubauer du nombre de parasites vivants par puits. On détermine ainsi, pour chaque quinoléine substituée testée, la concentration inhibant 50 % des parasites ($CI_{50}$).

### e) Essais sur la réplication du VIH-1

Des cellules CEM4fx sont infectées *de novo* par le clone moléculaire pLN4-3 du VIH-1 et traitées simultanément par les quinoléines substituées à tester préalablement dissoutes dans du DMSO. Le virus se multiplie pendant trois jours, puis la présence de virions infectieux est détectée dans le surnageant de culture (milieu de RPMI) en réinfectant des cellules dites P4 qui possèdent un gène rapporteur s'exprimant lorsqu'elles sont infectées par le virus. La charge virale est quantifiée après 72 heures de culture par deux méthodes : l'estimation de la charge virale par infection de cellules HeLa-βgal $CD_4^+$ et la détermination de la quantité de protéine virale p24 par un test ELISA.

Par ailleurs, la cytotoxicité des quinoléines vis-à-vis des cellules hôtes est mesurée par un test de biotransformation du MTT [bromure de 3-(4,(-diméthylthiazol-2-yl)-2,5-diphényltétrazolium]. Tous les tests sont réalisés en triplicate.

### 3) Résultats :

Les résultats obtenus avec les quinoléines substituées, affectées des n° 1, 2, 4, 6 à 11, 13 à 20, 23 à 34, 37 et 38 dans le Tableau I, sont présentés dans le Tableau II ci-après pour ce qui concerne leur activité sur les amastigotes de *Leishmania amazonensis,* de *Leishmania donovani* et de *Leishmania infantum,* ainsi que leur cytotoxicité vis-à-vis des macrophages.

TABLEAU II

| **Quinoléines** | ***L. amazonensis*** $CI_{100}$(µg/ml) | **Cytotoxicité sur** les macrophages (µg/ml) | *L.* ***donovani*** $CI_{50}$(µg/ml) | ***L. infantum*** (IPZ229/1/89) $CI_{50}$ (µg/ml) | ***L. infantum*** (MHOM/MA (BE) 67) $CI_{50}$ (µM) |
|---|---|---|---|---|---|
| 1 | 0,78125 | 6,25 | | | >32 |
| 2 | 3,12 | 6,25 | 1,2-2,8 | 1,3 | >32 |
| 4 | 25 | 25 | | | |
| 5 | | | | | 32 |
| 6 | 6,25 | 25 | | | |
| 7 | 12,5 | 25 | | | |

(suite)

| **Quinoléines** | ***L. amazonensis*** $CI_{100}$(µg/ml) | **Cytotoxicité sur** les macrophages (µg/ml) | *L.* ***donovani*** $CI_{50}$(µg/ml) | ***L. infantum*** (IPZ229/1/89) $CI_{50}$ (µg/ml) | ***L. infantum*** (MHOM/MA (BE) 67) $CI_{50}$ (µM) |
|---|---|---|---|---|---|
| 8 | 12,5 | 12,5 | | | |
| 9 | 0,781 | 1,56 | | | |
| 10 | 1,56 | 6,25 | | | 3 |
| 11 | 50 | 50 | | | 14 |
| 13 | 25 | 25 | | | |
| 14 | 25 | 100 | | | |
| 16 | 10-20 | | | | |
| 17 | | | | | 16 |
| 18 | 12,5 | 50 | | | >32 |
| 19 | 12,5 | 12,5 | | | |
| 20 | >50 | > 100 | | | 16 |
| 23 | 12,5 | 50 | 3,3-30 | 0,4-2,2 | 26 |
| 24 | 10-25 | | | | |
| 25 | 12,5 | 25 | | | 16 |
| 26 | 12,5 | 1,56 | 1,7 | 10,9 | 10 |
| 27 | 50 | | | | 10 |
| 28 | 12,5 | 12,5 | | | >32 |
| 29 | 0,78125 | 3,12 | | | 2 |
| 30 | 12,5 | 25 | | | >32 |
| 31 | 25 | 25 | | | |
| 32 | 12,5 | 12,5 | | | |
| 33 | 10-25 | | | | |
| 34 | 50 | 25 | | | 2 |
| 35 | | | | | 10 |
| 37 | 50-25 | | | 0,3-5,8 | 17 |
| 38 | 25 | 25 | | | |

Un composé est considéré comme présentant une activité intéressante sur les amastigotes de *Leishmania amazonensis* lorsqu'il présente une $CI_{100}$ inférieure ou égale à 25 µg/ml. Il est, par ailleurs, considéré comme présentant une activité intéressante sur les amastigotes de *Leishmania donovani* et *infantum* lorsqu'il présente une $CI_{50}$ inférieure ou égale à 12,5 µg/ml.

Les résultats obtenus avec les quinoléines substituées, affectées des n° 2, 6, 9 à 11, 13, 15, 17, 18, 20 à 23, 25, 28, 29 et 34 à 37 dans le Tableau I, sont présentés dans le Tableau III ci-après pour ce qui concerne leur activité sur les amastigotes de *Trypanosoma cruzi* et les formes circulantes de *Trypanosoma brucei.*

TABLEAU III

| **Quinoléines** | ***T. cruzi*** (amastigotes) $CI_{50}$ (µM) | ***T. brucei*** $CI_{50}$ (µM) |
|---|---|---|
| 1 | 8 | |
| 2 | 12 | 13 |

(suite)

| Quinoléines | **T. cruzi** (amastigotes) $CI_{50}$ (μM) | **T. brucei** $CI_{50}$ (μM) |
|---|---|---|
| 5 | >32 | |
| 6 | >32 | 17 |
| 9 | 5 | 19 |
| 10 | 4 | >32 |
| 11 | 19 | >32 |
| 13 | >32 | 10 |
| 15 | >32 | 16 |
| 17 | >32 | 3 |
| 18 | >32 | 16 |
| 20 | 19 | 13 |
| 21 | >32 | 14 |
| 22 | 16 | 16 |
| 23 | 15 | 16 |
| 25 | 11 | 4 |
| 26 | 8 | |
| 27 | 25 | |
| 28 | >32 | 5 |
| 29 | <0.5 | 1 |
| 30 | 26 | |
| 34 | 15 | 13 |
| 35 | >32 | 24 |
| 36 | 21 | 4 |
| 37 | >32 | 13 |

Un composé est considéré comme présentant une activité intéressante sur les amastigotes de *Trypanosoma cruzi* lorsqu'il présente une $CI_{50}$ inférieure ou égale à 20 μM. Il est, par ailleurs, considéré comme présentant une activité intéressante sur les formes circulantes de *Trypanosoma brucei* lorsqu'il présente une $CI_{50}$ inférieure ou égale à 5 μM.

Les résultats obtenus avec les quinoléines substituées, affectées des n° 9, 20 et 26 dans le Tableau I, sont présentés dans le Tableau IV ci-après pour ce qui concerne leur cytotoxicité vis-à-vis des cellules CEM4fx et leur activité sur la réplication du VIH-1 dans ces cellules.

TABLEAU IV

| Quinoléines | **Cytotoxicité** CIso (μM) | **Activité antirétrovirale** $CI_{50}$ (μM) |
|---|---|---|
| 9 | 2 | 0,6 |
| 20 | 9 | 2 |
| 26 | 3 | 0,9 |

Comme le montrent les Tableaux II et III, l'activité antiprotozoaire des quinoléines substituées varie, dans la plupart des cas, en fonction des spécificités des parasites, voire des souches, sur lesquelles elles sont testées. Ainsi, par exemple, la quinoléine affectée du n° 23 présente une activité marquée sur *Leishmania amazonensis, Leishmania donovani* et sur la souche IPZ229/1/89 de *Leishmania infantum,* alors que son activité se révèle être moins intéressante sur la souche MHOM/MA(BE)67 de *Leishmania infantum,* ainsi que sur *Trypanosoma cruzi* et *Trypanosoma brucei.*

De manière similaire, la quinoléine substituée affectée du n° 25 montre une activité intéressante sur *Leishmania amazonensis, Trypanosoma cruzi* et *Trypanosoma brucei,* alors qu'elle apparaît moins active sur *Leishmania infantum.*

Aussi, l'homme du métier choisira-t-il, en fonction des parasites responsables de la co-infection qu'il souhaite traiter, la quinoléine substituée qui lui paraîtra la plus appropriée. Il pourra également associer deux ou plusieurs quinoléines substituées différentes de façon à optimiser la réponse thérapeutique ou à élargir le spectre d'action thérapeutique.

**II- ACTIVITE BIOLOGIQUE *IN VIVO* DES OUINOLEINES SUBSTITUEES**

**a) Essais effectués sur des animaux de laboratoire infectés avec la leishmaniose cutanée *(Leishmania amazonensis):***

1/ Animaux de laboratoire

Les animaux d'expérimentation sont des souris Balb/c de 18-24 g mâles ou femelles âgées de 6-8 semaines environ, élevés ensuite pour la reproduction à l'animalerie de l'Instituto de Investigaciones en Ciencias de la Salud (IICS), Asuncion, Paraguay. Des hamsters dorés (*Mesocricetus auratus*) sont employés pour l'entretien des souches et comme réservoirs de parasites *L. amazonensis.*

2/ Parasites

Les souris Balb/c sont infectées par des parasites au stade amastigote de *Leishmania amazonensis* de la souche de référence IFLA/BR/67/PH8 par voie sous cutanée au niveau du coussinet ventral de la patte arrière droite, la patte gauche restant comme témoin. La quantité d'amastigotes inoculée est de 2x $10^6$ amastigotes dans 50 $\mu$l de solution saline phosphatée (PBS) pour *L. amazonensis.*

3/ Traitement de la leishmaniose cutanée

Les traitements commencent une fois que l'infection est bien établie, soit en général 5 à 6 semaines après l'inoculation des amastigotes de *L. amazonensis.* Le médicament de référence choisi est le Glucantime® ou l'antimoniate de méglumine d'Aventis (France).

Les traitements s'effectuent de la manière suivante (8 souris par groupe):

- le Glucantime® est administré par voie sous-cutanée à la concentration de 100 mg/kg ou 28 mg de $Sb^V$/kg à raison d'une injection quotidienne pendant 15 jours .
- les quinoléines sont administrées par voie orale à 25 mg/kg/jour pendant 15 jours, en deux fois, le matin et à midi,

Les quinoléines ou l'antimoniate de N-méthyl glucamine (Glucantime®) sont dissous dans une goutte de Tween 80 complétée avec une solution saline phosphatée pour obtenir la concentration désirée, ensuite 50 $\mu$l de la solution obtenue sont administrés. Les souris témoins non-traitées reçoivent 50 $\mu$l d'une solution de Tween 80 avec du PBS.

4/ Paramètres étudiés

Mesure de la lésion : Les diamètres des lésions de la patte infectée et de la patte témoin sont mesurés à l'aide d'un micromètre gradué en 1/10 mm une fois par semaine pendant la durée de l'expérimentation. Les premières mesures commencent 48 heures avant l'inoculation des parasites. Le calcul de la différence des deux mesures donne l'épaisseur de la lésion.

Numération des formes amastigotes dans la lésion : Après l'arrêt des traitements, les animaux sont sacrifiés, puis les lésions sont recueillies dans une boîte de Pétri, pesées, coupées en morceau et broyées à l'aide d'un broyeur de Potter de 10 ml avec 5 ml de milieu de culture RPMI (Rosewall Park Medecine Institute, USA, Gibco). Les 5 ml de purée obtenus sont centrifugés deux fois. La couche renfermant les amastigotes fait l'objet d'un comptage du nombre de parasites à l'aide d'une cellule de Neubauer. Cinq énumérations sont effectuées sur chaque couche d'amastigotes. Le nombre de formes amastigotes est déterminé à l'aide de la formule de Stauber qui prend en compte le poids de la lésion:

$$A/N \times P \times \text{coefficient de la cellule de Neubauer}$$

A = nombre d'amastigotes, N = nombre de noyaux
P = poids de la lésion

Les résultats sont exprimés par le calcul de la moyenne arithmétique des diamètres des lésions mesurés une fois par semaine, et en fin de protocole après sacrifice des souris, des poids des lésions et du nombre d'amastigotes. Un test de Student (t-test) est employé pour l'analyse statistique de toutes les données. La comparaison s'effectue entre groupe "témoins" avec un groupe "traitement" par paire. L'obtention d'une valeur de P< 0,05 est considérée comme statistiquement significatif.

La figure 1 illustre l'effet des traitements avec le Glucantime (28 mg de $Sb^V$ /kg/jour) administré par voie sous cutanée, le E-l-(2-quinolyl)-butène (6) et le E-3-(2-quinolyl)-2-propénate de méthyle (23) administrés par voie orale à 25 mg/kg/jour pendant 15 jours sur des souris Balb/c infectées (*n*=8) avec *L. amazonensis*

Le tableau V résume l'effet des traitements avec le Glucantime, le E-1-(2-quinolyl)-butène (6) et le E-3-(2-quinolyl)-2-propénate de méthyle (23) sur des souris Balb/c infectées (*n*=8) avec *L. amazonensis*

**Tableau V**

| Composé | Voie d'administration | Poids des lésions (g) (moyenne ± déviation standard) | % réduction du nombre de parasites dans la lésion | Moyenne du nombre de parasites dans la lésion par gramme |
|---|---|---|---|---|
| Témoins sans traitement | | 0,061±0,038 | - | $2.5 \times 10^7$ |
| Meglumine antimonate | sous-cutanée | 0,012±0,010 | -97 % * | $7,2 \times 10^5$ |
| E-1-(2-quinolyl)-butène (6) | orale | 0,101±0,100 | -73 % | 6,9 x 106 |
| E-3-(2-quinolyl)-2-propénate de méthyle (23) | orale | 0,059±0,010 | - 88 %** | $3,1 \times 10^6$ |

Test de Student :
**P* = 0,02, ** *P* = 0,05 (versus groupe non traité)

**b) Essais effectués sur des animaux de laboratoire infectés avec la leishmaniose viscérale *(Leishmania infantum) :***

1/ Animaux de laboratoire et parasites

Les animaux d'expérimentation sont des souris Balb/c de 18-24 g mâles ou femelles âgées de 6-8 semaines environ élevées à l'animalerie de l'Instituto de Insvestigaciones en Ciencias de la Salud, Asuncion (Paraguay). Les souris Balb/c sont infectées avec des parasites au stade promastigote de *Leishmania infantum* (Référence : MHOM/FR/91/LEM2259V), une souche isolée d'un patient porteur du SIDA. Cette souche est maintenue dans le milieu de culture Schneider'drosophila (Gibco, USA) supplémentée avec 20 % de sérum de veau foetal. Les promastigotes infectieux sont cultivés pendant 7 jours, puis comptés. Chaque souris est ensuite infectée par voie intraveineuse avec 0,2 ml de milieu de culture contenant $10^7$ promastigotes.

2/ Traitement de la leishmaniose viscérale

Les souris infectées sont réparties au hasard en plusieurs groupes de 8 souris, puis traitées une semaine après l'infection parasitaire de la manière suivante :

- le Glucantime® est administré par voie sous-cutanée à 100 mg/kg/jour ou 28 mg de $Sb^V$/kg/jour à raison d'une injection quotidienne pendant 10 jours .
- les quinoléines sont administrées par voie orale à 25 mg/kg/jour pendant 10 jours, répartie en deux fois, le matin et à midi.

Les quinoléines ou l'antimoniate de N-méthyl glucamine (Glucantime®) sont dissous dans une goutte de Tween 80 complétée avec une solution saline phosphatée (PBS) pour obtenir la concentration désirée, 50 μl de la solution

obtenue sont administrés. Les souris témoins non-traitées reçoivent 50 µl d'une solution de Tween 80 avec du PBS.

' 3/ Paramètres étudiés

Numération des formes amastigotes dans le foie et la rate.

Après l'arrêt des traitements et sacrifice des animaux, les viscères, foie et rate, sont pesés et recueillis dans une boîte de Pétri, Avec chaque organe on procède à plusieurs appositions sur une lame de verre dégraissées qui, fixées puis colorées par la technique de May-Grünwald-Giemsa, permettent de déterminer le nombre de formes amastigotes pour 500 cellules nuclées. Ensuite, les organes sont coupés en morceau et broyés à l'aide d'un broyeur de Potter de 10 ml avec 5 ml de milieu de culture RPMI. Les 5 ml de purée obtenus sont centrifugés deux fois. La couche renfermant les amastigotes fait l'objet d'un comptage du nombre de parasites à l'aide d'une cellule de Neubauer. Cinq énumérations sont effectuées sur chaque couche d'amastigotes. Le nombre de formes amastigotes est déterminé à l'aide de la formule de Stauber qui prend en compte le poids de l'organe récupéré:

$$A/N \times P \times \text{coefficient de la cellule de Neubauer}$$

A = nombre d'amastigotes, N = nombre de noyaux
P = poids de la lésion

Un test de Student (*t*-test) est employé pour l'analyse statistique de toutes les données. La comparaison s'effectue entre groupe "témoins" avec un groupe "traitement" par paire. L'obtention d'une valeur de $P<0{,}05$ est considérée comme statistiquement significatif. Les résultats sont exprimés dans le tableau VI.

Le tableau VI illustre l'efficacité du 3-(2-quinolyl)-propénal (9), du 1-(2-quinolyl)-acétylène (1), du *E*-3-(2-quinolyl)-2-propénoate de méthyle (23) et du Glucantime sur des souris Balb/c (*n*= 8) infectées avec *Leishmania infantum* infectées avec *Leishmania infantum.*

**Tableau VI**

| Traitement | Poids du foie (g) | Nombre de parasites dans le foie par gramme | % de réduction du nombre de parasites dans le foie | Poids de la rate (g) | Nombre de parasites dans la rate par gramme | % de réduction du nombre de parasites dans la rate |
|---|---|---|---|---|---|---|
| Sans traitement | 0,93 | $4.5 \times 10^7$ | | 0,10 | $4{,}2 \times 10^6$ | |
| Glucantime | 1,10 | $1{,}6 \times 10^{7\,a}$ | - 65,3 | 0,11 | $4{,}4 \times 10^6$ , | + 6,0 |
| 3-(2-quinolyl)-propénal (9) | 0,93 | $3{,}8 \times 10^{6\,a,\,e}$ | - 91,6 | 0,11 | $2{,}2 \times 10^6\ b$ | -47,6 |
| 1-(2-quinolyl)-acétylène (1) | 0,97 | $1{,}8 \times 10^{7c}$ | - 60,0 | 0,11 | $2{,}2 \times 10^6\ b$ | - 47,6 |
| *E*-3-(2-quinolyl)-2propénoate de méthyle (23) | 1,01 | $1{,}8 \times 10^{7\,d}$ | - 60,0 | 0,10 | $1{,}5 \times 10^6\ c, f$ | - 64,3 |

Test de Student :
[a] *P*= 0,02 (versus groupe infecté sans traitement)
[b] *P*= 0,01 (versus groupe infecté sans traitement)
[c] *P*= 0,03 (versus groupe infecté sans traitement)
[d] *P*= 0,04(versus groupe infecté sans traitement)
[e] *P*= 0,002(versus groupe traité avec le Glucantime)
[f] *P*= 0,03(versus groupe traité avec le Glucantime)

## Revendications

**1.** Utilisation d'au moins une quinoléine répondant à la formule générale (I) :

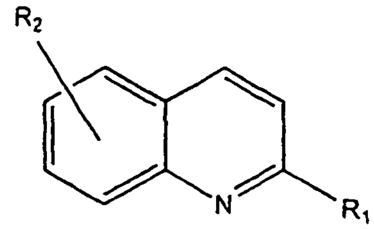

(I)

dans laquelle :

- $R_1$ représente :

  • un atome d'hydrogène, un groupe alkyle en $C_1$ à $C_{15}$ un groupe alkényle en $C_2$ à $C_{15,}$ un groupe alkynyle en $C_2$ à $C_{15,}$ un groupe formyle ou un groupe hétéroaryle, ce dernier étant éventuellement substitué par un ou plusieurs groupes hydroxyle ; ou bien
  • un groupe alkyle en $C_1$ à $C_{15}$ ou alkényle en $C_2$ à $C_7$ portant au moins un substituant choisi parmi l'oxygène, les halogènes et les groupes hydroxyle, formyle, carboxyle, aryloxycarbonyle, alkyloxycarbonyle en $C_2$ à $C_8$, alkényioxycarbonyle en $C_3$ à $C_9$, nitrile, amine, alcoxy en $C_1$ à $C_7$, phénoxy, cycloalkyle en $C_3$ à $C_6$, aryle, hétéroaryle, hétéroaryloxy, arylsulfone, alkylsulfone en $C_1$ à $C_7$, thioalkyle en $C_1$ à $C_7$ et aminoalkyle en $C_1$ à $C_7$ ; ou bien
  • un groupe alkynyle en $C_2$ à $C_7$ portant au moins un substituant choisi parmi l'oxygène, les halogènes et les groupes hydroxyle, formyle, carboxyle, aryloxycarbonyle, alkyloxycarbonyle en $C_2$ à $C_8$, alkényloxycarbonyle en $C_3$ à $C_9$, nitrile, aryle, hétéroaryle, arylsulfone, alkylsulfone en $C_1$ à $C_7$, thioalkyle en $C_1$ à $C_7$ et aminoalkyle $C_1$ à $C_7$ ; ou bien
  • un groupe alkényle ou alkynyle en $C_2$ à $C_{15}$ substitué par au moins un groupe trialkylsilyle en $C_1$ à $C_7$ ; tandis que

- $R_2$, qui peut être en position 3, 6 ou 8 sur le cycle quinoléique, représente un atome d'hydrogène ou d'halogène, un groupe hydroxyle, formyle, carboxyle, alkyle en $C_1$ à $C_7$, alcoxy en $C_1$ à $C_7$, amine, alkylamide en $C_1$-$C_{10}$, alkényle en $C_2$ à $C_7$ éventuellement substitué par un ou plusieurs groupes alcoxy en $C_1$ à $C_7$, ou encore un groupe alkynyle en $C_2$ à $C_{10'}$ ce dernier étant éventuellement substitué par un groupe hétéroaryle ;

ou l'un de ses sels pharmaceutiquement acceptables, à la condition toutefois que $R_1$ et $R_2$ ne soient pas tous deux un atome d'hydrogène, pour la préparation d'un médicament pour traiter les co-infections à protozoaires et à rétrovirus. '

**2.** Utilisation selon la revendication 1, dans laquelle $R_1$ est différent d'un atome d'hydrogène.

**3.** Utilisation selon la revendication 1 ou la revendication 2, dans laquelle $R_1$ représente un groupe alkényle ou alkynyle porteur ou non de substituants.

**4.** Utilisation selon l'une quelconque des revendications précédentes, dans laquelle :

- $R_1$ représente :

  • un groupe alkényle ou alkynyle en $C_2$ à $C_{15}$ ; ou bien
  • un groupe alkényle en $C_2$ à $C_7$ portant au moins un substituant choisi parmi l'oxygène, les halogènes et les groupes hydroxyle, formyle, carboxyle, alkyloxycarbonyle en $C_2$ à $C_8$, nitrile, amine, alcoxy en $C_1$ à $C_7$, phénoxy, cycloalkyle en $C_3$ à $C_6$, aryle, hétéroaryle, hétéroaryloxy, arylsulfone, alkylsulfone en $C_1$à $C_7$, thioalkyle en $C_1$ à $C_7$, aminoalkyle en $C_1$ à $C_7$ et triméthylsilyle ; ou bien
  • un groupe alkynyle en $C_2$ à $C_7$ portant au moins un substituant choisi parmi l'oxygène, les halogènes et

les groupes hydroxyle, formyle, carboxyle, alkyloxycarbonyle en $C_2$ à $C_8$, nitrile, aryle, hétéroaryle, arylsulfone, alkylsulfone en $C_1$ à $C_7$, thioalkyle en $C_1$ à $C_7$, aminoalkyle $C_1$ à $C_7$ et triméthylsilyle ; tandis que

- $R_2$ représente un atome d'hydrogène, un groupe hydroxyle ou un groupe alkyle en $C_1$ à $C_7$.

**5.** Utilisation selon l'une quelconque des revendications précédentes, dans laquelle $R_1$ représente un groupe alkényle ou alkynyle en $C_2$ à $C_7$ substitué par un plusieurs atomes d'halogène, notamment de chlore, de brome ou de fluor.

**6.** Utilisation selon l'une quelconque des revendications précédentes, dans laquelle le médicament est destiné à traiter une co-infection induite par un ou plusieurs protozoaires appartenant aux genres *Leishmania, Trypanosoma, Plasmodium, Toxoplasma, Pneumocystis* et *Schistosomia* et par un rétrovirus du type HIV ou HTLV-1.

**7.** Utilisation selon la revendication 6, dans laquelle le médicament est destiné à traiter une co-infection *Leishmania*lHIV.

**8.** Quinoléine répondant à la formule générale (I) selon la revendication 1, **caractérisée en ce que** :

- soit $R_1$ représente un groupe alkyle en $C_1$ à $C_7$, un groupe alkényle en $C_2$ à $C_7$, ou un groupe alkényle en $C_2$ à $C_7$ portant au moins un substituant choisi parmi les groupes aryle, auquel cas $R_2$, qui peut être en position 3, 6 ou 8 du cycle quinoléique, représente un groupe alkényle en $C_3$ à $C_7$ substitué par un ou plusieurs groupes alcoxy en $C_1$ à $C_7$, ou un groupe alkynyle en $C_2$ à $C_{10}$ substitué par un groupe hétéroaryle ;
- soit $R_1$ représente un groupe alkyle en $C_1$ à $C_7$ portant au moins un substituant choisi parmi les groupes hydroxyle, amine, alcoxy en $C_1$ à $C_4$ et aminoalkyle en $C_1$ à $C_4$, ou $R_1$ représente un groupe alkényle en $C_2$ à $C_7$ portant au moins un substituant choisi parmi les groupes hydroxyle, amine, alcoxy en $C_1$ à $C_4$ et aminoalkyle en $C_1$ à $C_4$, auquel cas $R_2$, qui peut être en position 3, 6 ou 8 du cycle quinoléique, représente un groupe alkényle en $C_3$ à $C_7$ substitué par un ou plusieurs groupes alcoxy en $C_1$ à $C_7$, ou un groupe alkynyle en $C_2$ à $C_{10}$, ce dernier étant éventuellement substitué par un groupe hétéroaryle ;
- soit $R_1$ représente :

  • un groupe méthyle ou éthyle portant au moins un substituant choisi parmi les halogènes ;

  auquel cas $R_2$, qui peut être en position 3, 6 ou 8 du cycle quinoléique, représente un groupe alcoxy en $C_1$ à $C_7$, amine, alkylamide en $C_1$-$C_{10}$, alkényle en $C_2$ à $C_7$ substitué par un ou plusieurs groupes alcoxy en $C_1$ à $C_7$, ou encore un groupe alkynyle en $C_2$ à $C_{10}$, substitué par un groupe hétéroaryle ;
- soit $R_1$ représente :

  un radical 2-pyridyle

  auquel cas $R_2$, qui peut être en position 3, 6 ou 8 du cycle quinoléique, représente un atome d'hydrogène ou d'halogène, formyle, carboxyle, alkyle en $C_1$ à $C_7$, alcoxy en $C_1$ à $C_7$, amine, alkylamide en $C_1$-$C_{10}$, alkényle en $C_2$ à $C_7$ éventuellement substitué par un ou plusieurs groupes alcoxy en $C_1$ à $C_7$, ou encore un groupe alkynyle en $C_2$ à $C_{10}$, ce dernier étant éventuellement substitué par un groupe hétéroaryle ;
- soit $R_1$ représente :

  • un groupe alkyle en $C_8$ à $C_{15}$, portant éventuellement un substituant choisi parmi les groupes aryle; ou bien
  • un groupe alkényle en $C_8$ à $C_{15}$, un groupe alkényle en $C_2$ à $C_7$ portant au moins un substituant choisi parmi les arylsulfone; ou bien
  • un groupe alkynyle en $C_2$ à $C_{15}$, un groupe alkynyle en $C_2$ à $C_7$ portant au moins un substituant choisi parmi les groupes aryle et arylsulfone ;

  auquel cas $R_2$, qui peut être en position 3, 6 ou 8 du cycle quinoléique, représente un groupe choisi parmi un alcoxy en $C_1$ à $C_7$, une amine, un alkylamide en $C_1$-$C_{10}$, un alkényle en $C_2$ à $C_7$ éventuellement substitué par un ou plusieurs groupes alcoxy en $C_1$ à $C_7$, ou encore un groupe alkynyle en $C_2$ à $C_{10}$ substitué par un groupe hétéroaryle ;
- soit $R_1$ représente :

  • un atome d'hydrogène, un groupe formyle, un groupe hétéroaryle éventuellement substitué par un ou plusieurs groupes hydroxyle , à l'exception du radical 2-pyridyl ; ou bien
  • un groupe alkyle en $C_1$ à $C_{15}$ portant au moins un substituant choisi parmi l'oxygène et les groupes

formyle, carboxyle, aryloxycarbonyle, alkyloxycarbonyle en $C_2$ à $C_8$, alkényloxycarbonyle en $C_3$ à $C_9$, nitrile, phénoxy, cycloalkyle en $C_3$ à $C_6$, hétéroaryloxy, arylsulfone, alkylsulfone en $C_1$ à $C_7$ et thioalkyle en $C_1$ à $C_7$, un groupe alkyle en $C_1$ à $C_7$ portant au moins un substituant choisi parmi les groupes alcoxy en $C_5$ à $C_7$ et aminoalkyle en $C_5$ à $C_7$, un groupe alkyle en $C_3$ à $C_{15}$ portant au moins un substituant choisi parmi les halogènes, un groupe alkyle en $C_6$ à $C_{15}$ substitué par au moins un groupe hétéroaryle, un groupe alkyle en $C_8$ à $C_{15}$ portant au moins un substituant choisi parmi les groupes hydroxyle, amine, alcoxy en $C_1$ à $C_7$ et aminoalkyle en $C_1$ à $C_7$ ; ou bien

• un groupe alkényle en $C_2$ à $C_7$ portant au moins un substituant choisi parmi l'oxygène, les halogènes et les groupes carboxyle, aryloxycarbonyle, alkyloxycarbonyle en $C_2$ à $C_8$, alkényloxycarbonyle en $C_3$ à $C_9$, nitrile, phénoxy, cycloalkyle en $C_3$ à $C_6$, hétéroaryloxy, alkylsulfone en $C_1$ à $C_7$, thioalkyle en $C_1$ à $C_7$, alcoxy en $C_1$ à $C_7$ et aminoalkyle en $C_5$ à $C_7$ ; un groupe alkényle en $C_3$ à $C_7$ substitué par un groupe hétéroaryle ; ou bien

• un groupe alkynyle en $C_2$ à $C_7$ portant au moins un substituant choisi parmi l'oxygène, les halogènes et les groupes hydroxyle, formyle, carboxyle, aryloxycarbonyle, alkyloxycarbonyle en $C_2$ à $C_8$, alkényloxycarbonyle en $C_3$ à $C_9$, nitrile, hétéroaryle, alkylsulfone en $C_1$ à $C_7$, thioalkyle en $C_1$ à $C_7$ et aminoalkyle $C_1$ à $C_7$ ; ou bien encore

• un groupe alkényle ou alkynyle en $C_2$ à $C_{15}$ substitué par au moins un groupe trialkylsilyle en $C_1$ à $C_7$ ;

auquel cas $R_2$, qui peut être en position 3, 6 ou 8 du cycle quinoléique, représente un atome d'hydrogène ou d'halogène, un groupe hydroxyle, formyle, carboxyle, alkyle en $C_1$ à $C_7$, alcoxy en $C_1$ à $C_7$, amine, alkylamide en $C_1$-$C_{10}$, alkényle en $C_2$ à $C_7$ éventuellement substitué par un ou plusieurs groupes alcoxy en $C_1$ à $C_7$, ou encore un groupe alkynyle en $C_2$ à $C_{10}$, ce dernier étant éventuellement substitué par un groupe hétéroaryle ; ou l'un de ses sels pharmaceutiquement acceptables à la condition toutefois que $P_1$ et $P_2$ ne soient pas tous deux un atome d'hydrogène, pour l'utilisation comme médicaments.

**9.** Quinoléine selon la revendication 8, **caractérisée en ce que** $R_1$ représente un groupe alkényle ou alkynyle porteur ou non de substituants.

**10.** Quinoléine selon la revendication 8 ou la revendication 9, **caractérisée en ce que** :

- soit $R_1$ représente un groupe un groupe alkényle en $C_2$ à $C_7$ éventuellement substitué par un ou plusieurs groupes choisis parmi les groupes hydroxyle, amine, aryle, alcoxy en $C_1$ à $C_4$ et aminoalkyle en $C_1$ à $C_4$, auquel cas $R_2$ représente un groupe alkényle en $C_3$ à $C_7$ substitué par un ou plusieurs groupes alcoxy en $C_1$ à $C_7$, ou un groupe alkynyle en $C_2$ à $C_{10}$, ce dernier étant éventuellement substitué par un groupe hétéroaryle;
- soit $R_1$ représente :

• un groupe alkényle en $C_2$ à $C_7$ portant au moins un substituants choisi parmi l'oxygène, les halogènes et les groupes carboxyle, alkyloxycarbonyle en $C_2$ à $C_8$, nitrile, phénoxy, cycloalkyle en $C_3$ à $C_6$, hétéroaryloxy, alkylsulfone en $C_1$ à $C_7$, thioalkyle en $C_1$ à $C_7$, alcoxy en $C_5$ à $C_7$ et aminoalkyle en $C_5$ à $C_7$ ; un groupe alkényle en $C_3$ à $C_7$ substitué par un groupe hétéroaryle ; ou bien

• un groupe alkynyle en $C_2$ à $C_7$ portant au moins un substituant choisi parmi l'oxygène, les halogènes et les groupes hydroxyle, formyle, carboxyle, alkyloxycarbonyle en $C_2$ à $C_8$, nitrile, hétéroaryle, alkylsulfone en $C_1$ à $C_7$, thioalkyle en $C_1$ à $C_7$ et aminoalkyle $C_1$ à $C_7$ ; ou bien encore

• un groupe alkényle ou alkynyle en $C_2$ à $C_{15}$ substitué par au moins un groupe trialkylsilyle en $C_1$ à $C_7$ ;

auquel cas $R_2$ représente un atome d'hydrogène, un groupe hydroxyle ou un groupe alkyle en $C_1$ à $C_7$.

**11.** Quinoléine selon l'une quelconque des revendications 8 à 10, **caractérisée en ce que** $R_1$ représente un groupe alkényle ou alkynyle en $C_2$ à $C_7$ substitué par un plusieurs atomes d'halogène, en particulier de chlore, de brome ou de fluor.

**12.** Quinoléine, **caractérisée en ce qu'**elle répond à la formule générale (I) selon la revendication 1 dans laquelle :

- soit $R_1$ représente un groupe cyclopropyl-hydroxyméthyle, 4-chloro-but-3-en-1-yn-1-yle, hept-1-en-1-yle, 2-bromo-éthényle, 2-bromoéthynyle, 2-bromo-2-fluoro-éthényle, 4-méthylcarboxylate-but-1,3-dien-1-yle, dec-1-yn-1-yle, 2-(2-quinoléyl)-éthényle, 2-(triméthylsilyl-éthynyl)-4-triméthylsilyl-but-1-en-3-yn-1-yle, auquel cas $R_2$ représente un atome d'hydrogène ;
- soit $R_1$ représente un groupe prop-1-en-1-yle, auquel cas $R_2$ représente un groupe méthyle en position 6 ou

un groupe hydroxyle en position 8 du cycle quinoléique ;
- soit $R_1$ représente un groupe 2-hydroxypropyle, auquel cas $R_2$ représente un groupe hydroxyle en position 8 du cycle quinoléique ;
- soit encore $R_1$ représente un groupe 2-méthylcarboxylate-éthényle, auquel cas $R_2$ représente un groupe méthyle en position 6 du cycle quinoléique.

**13.** Composition pharmaceutique, **caractérisée en ce qu'**elle comprend, à titre de principe actif, au moins une quinoléine selon la revendication 12.

## Claims

**1.** Use of at least one quinoline corresponding to general formula (I):

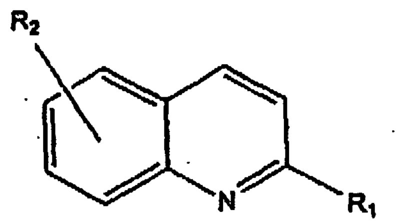

(I)

wherein:

- $R_1$ represents:

  - a hydrogen atom, a $C_1$-$C_{15}$ alkyl group, a $C_2$-$C_{15}$ alkenyl group, a $C_2$-$C_{15}$ alkynyl group, a formyl group or a heteroaryl group, the latter optionally being substituted by one or more hydroxyl groups; or
  - a $C_1$-$C_{15}$ alkyl group or $C_2$-$C_7$ alkenyl group having at least one substituent selected from among oxygen, the halogens and the hydroxyl, formyl, carboxyl, aryloxycarbonyl, $C_2$-$C_8$ alkyloxycarbonyl, $C_3$-$C_9$ alkenyloxycarbonyl, nitrile, amine, $C_1$-$C_7$ alkoxy, phenoxy, $C_3$-$C_6$ cycloalkyl, aryl, heteroaryl, heteroaryloxy, arylsulphonyl, $C_1$-$C_7$ alkylsulphonyl, $C_1$-$C_7$ thioalkyl and $C_1$-$C_7$ aminoalkyl groups; or
  - a $C_2$-$C_7$ alkynyl group having at least one substituent selected from among oxygen, the halogens and the hydroxyl, formyl, carboxyl, aryloxycarbonyl, $C_2$-$C_8$ alkyloxycarbonyl, $C_3$-$C_9$ alkenyloxycarbonyl, nitrile, aryl, heteroaryl, arylsulphonyl, $C_1$-$C_7$ alkylsulphonyl, $C_1$-$C_7$ thioalkyl and $C_1$-$C_7$ aminoalkyl groups; or
  - a $C_2$-$C_{15}$ alkenyl or alkynyl group substituted by at least one $C_1$-$C_7$ trialkylsilyl group; whereas

- $R_2$, which may be in the 3, 6 or 8 position on the quinoline ring, represents a hydrogen or halogen atom, a hydroxyl, formyl, carboxyl, $C_1$-$C_7$ alkyl, $C_1$-$C_7$ alkoxy, amine, $C_1$-$C_{10}$ alkylamide, $C_2$-$C_7$alkenyl group optionally substituted by one or more $C_1$-$C_7$ alkoxy groups, or a $C_2$-$C_{10}$ alkynyl group, the latter optionally being substituted by a heteroaryl group;

or one of the pharmaceutically acceptable salts thereof, with the condition that $R_1$ and $R_2$ do not both represent a hydrogen atom, for the preparation of a medicament for treating protozoan and retrovirus co-infections.

**2.** Use according to claim 1, wherein $R_1$ is other than a hydrogen atom.

**3.** Use according to claim 1 or claim 2, wherein $R_1$ represents a substituted or unsubstituted alkenyl or alkynyl group.

**4.** Use according to any one of the preceding claims, where:

- $R_1$ represents:

  - a $C_2$-$C_{15}$ alkenyl or alkynyl group; or

• a $C_2$-$C_7$ alkenyl group having at least one substituent selected from among oxygen, the halogens and the hydroxyl, formyl, carboxyl, $C_2$-$C_8$ alkyloxycarbonyl, nitrile, amine, $C_1$-$C_7$ alkoxy, phenoxy, $C_3$-$C_6$ cycloalkyl, aryl, heteroaryl, heteroaryloxy, arylsulphonyl, $C_1$-$C_7$ alkylsulphonyl, $C_1$-$C_7$ thioalkyl, $C_1$-$C_7$ aminoalkyl and trimethylsilyl groups ; or
• a $C_2$-$C_7$ alkynyl group having at least one substituent selected from among oxygen, the halogens and the hydroxyl, formyl, carboxyl, $C_2$-$C_8$ alkyloxycarbonyl, nitrile, aryl, heteroaryl, arylsulphonyl, $C_1$-$C_7$ alkylsulphonyl, $C_1$-$C_7$ thioalkyl, $C_1$-$C_7$ aminoalkyl and trimethylsilyl groups; whereas

- $R_2$ denotes a hydrogen atom, a hydroxyl group or a $C_1$-$C_7$ alkyl group.

**5.** Use according to any one of the preceding claims, wherein $R_1$ denotes a $C_2$-$C_7$ alkenyl or alkynyl group substituted by one or more halogen atoms, notably chlorine, bromine or fluorine.

**6.** Use according to any one of the preceding claims, wherein the medicament is intended for treating a co-infection induced by one or more protozoa belonging to the genera *Leishmania, Trypanosoma, Plasmodium, Toxoplasma, Pneumocystis* and *Schistosomia* and by a retrovirus of the HIV or HTLV-1 type.

**7.** Use according to claim 6, wherein the medicament is intended for treating a *Leishmania*/HIV co-infection.

**8.** Quinoline corresponding to general formula (I) according to claim 1, **characterised in that**:

- either $R_1$ represents a $C_1$-$C_7$ alkyl group, a $C_2$-$C_7$ alkenyl group, or a $C_2$-$C_7$ alkenyl group carrying at least one substituent selected from among the aryl groups, in which case $R_2$, which may be in the 3, 6 or 8 position of the quinoline ring, represents a $C_3$-$C_7$ alkenyl group substituted by one or more $C_1$-$C_7$ alkoxy groups, or a $C_2$-$C_{10}$ alkynyl group substituted by a heteroaryl group;
- or $R_1$ denotes a $C_1$-$C_7$ alkyl group carrying at least one substituent selected from among the hydroxyl, amine, $C_1$-$C_4$ alkoxy and $C_1$-$C_4$ aminoalkyl groups, or $R_1$ represents a $C_2$-$C_7$ alkenyl group having at least one substituent selected from among the hydroxyl, amine, $C_1$-$C_4$ alkoxy and $C_1$-$C_4$ aminoalkyl groups, in which case $R_2$, which may be in the 3, 6 or 8 position of the quinoline ring, represents a $C_3$-$C_7$ alkenyl group substituted by one or more $C_1$-$C_7$ alkoxy groups, or a $C_2$-$C_{10}$ alkenyl group, the latter optionally being substituted by a heteroaryl group;
- or $R_1$ represents:

• a methyl or ethyl group having at least one substituent selected from among the halogens;

in which case $R_2$, which may be in the 3, 6 or 8 position of the quinoline ring, represents a $C_1$-$C_7$ alkoxy, amine, $C_1$-$C_{10}$ alkylamide, $C_2$-$C_7$ alkenyl group substituted by one or more $C_1$-$C_7$ alkoxy groups, or a $C_2$-$C_{10}$ alkenyl group substituted by a heteroaryl group;
- or $R_1$ represents :

a 2-pyridyl radical

in which case $R_2$, which may be in the 3, 6 or 8 position of the quinoline ring, represents a hydrogen or halogen atom, a formyl, carboxyl, $C_1$-$C_7$ alkyl, $C_1$-$C_7$ alkoxy, amine, $C_1$-$C_{10}$ alkylamide, $C_2$-$C_7$ alkenyl group optionally substituted by one or more $C_1$-$C_7$ alkoxy groups, or a $C_2$-$C_{10}$ alkynyl group, the latter optionally being substituted by a heteroaryl group;
- or $R_1$ denotes:

• a $C_8$ to $C_{15}$ alkyl group, optionally carrying a substituent selected from among the aryl groups; or
• a $C_8$-$C_{15}$ alkenyl group, a $C_2$-$C_7$ alkenyl group carrying at least one substituent selected from among the arylsulphonyls; or
• a $C_2$-$C_{15}$ alkynyl group, a $C_2$-$C_7$ alkynyl group having at least one substituent selected from among the aryl and arylsulphonyl groups;

in which case $R_2$, which may be in the 3, 6 or 8 position of the quinoline ring, represents a group selected from among a $C_1$-$C_7$ alkoxy, an amine, a $C_1$- $C_{10}$ alkylamide, a $C_2$-$C_7$ alkenyl optionally substituted by one or more $C_1$-$C_7$ alkoxy groups, or a $C_2$-$C_{10}$ alkynyl group substituted by a heteroaryl group;
- or $R_1$ represents:

• a hydrogen atom, a formyl group, a heteroaryl group optionally substituted by one or more hydroxyl groups, with the exception of the 2-pyridyl radical; or

• a $C_1$-$C_{15}$ alkyl group having at least one substituent selected from, among oxygen and the formyl, carboxyl, aryloxycarbonyl, $C_2$-$C_8$ alkyloxycarbonyl, $C_3$-$C_9$ alkenyloxycarbonyl, nitrile, phenoxy, $C_3$-$C_6$ cycloalkyl, heteroaryloxy, arylsulphonyl, $C_1$-$C_7$ alkylsulphonyl and $C_1$-$C_7$ thioalkyl groups, a $C_1$-$C_7$ alkyl group having at least one substituent selected from among the $C_5$-$C_7$ alkoxy and $C_5$-$C_7$ aminoalkyl groups, a $C_3$-$C_{15}$ alkyl group having at least one substituent selected from among the halogens, a $C_6$-$C_{15}$ alkyl group substituted by at least one heteroaryl group, a $C_8$-$C_{15}$ alkyl group having at least one substituent selected from among the hydroxyl, amine, $C_1$-$C_7$ alkoxy and $C_1$-$C_7$ aminoalkyl groups; or

• a $C_2$-$C_7$ alkenyl group having at least one substituent selected from among oxygen, the halogens and the carboxyl, aryloxycarbonyl, $C_2$-$C_8$ alkyloxycarbonyl, $C_3$-$C_9$ alkenyloxycarbonyl, nitrile, phenoxy, $C_3$-$C_6$ cycloalkyl, heteroaryloxy, $C_1$-$C_7$ alkylsulphonyl, $C_1$-$C_7$ thioalkyl, $C_1$-$C_7$ alkoxy and $C_5$-$C_7$ aminoalkylgroups; a $C_3$-$C_7$ alkenyl group substituted by a heteroaryl group; or

• a $C_2$-$C_7$ alkynyl group having at least one substituent selected from among oxygen, the halogens and the hydroxyl, formyl, carboxyl, aryloxycarbonyl, $C_2$-$C_8$ alkyloxycarbonyl, $C_3$-$C_9$ alkenyloxycarbonyl, nitrile, heteroaryl, $C_1$-$C_7$ alkylsulphonyl, $C_1$-$C_7$ thioalkyl and $C_1$-$C_7$ aminoalkyl groups ; or

• a $C_2$-$C_{15}$ alkenyl or alkynyl group substituted by at least one $C_1$-$C_7$ trialkylsilyl group;

in which case $R_2$, which may be in the 3, 6 or 8 position of the quinoline ring, represents a hydrogen or halogen atom, a hydroxyl, formyl, carboxyl, $C_1$-$C_7$ alkyl, $C_1$-$C_7$ alkoxy, amine, $C_1$-$C_{10}$ alkylamide, $C_2$-$C_7$ alkenyl group optionally substituted by one or more $C_1$-$C_7$ alkoxy groups, or a $C_2$-$C_{10}$ alkynyl group, the latter optionally being substituted by a heteroaryl group; or one of the pharmaceutically acceptable salts thereof, with the condition that $R_1$ and $R_2$ do not both represent a hydrogen atom, for use as medicaments.

**9.** Quinoline according to claim 8, **characterised in that** $R_1$ represents a substituted or unsubstituted alkenyl or alkynyl group.

**10.** Quinoline according to claim 8 or claim 9, **characterised in that**:

- either $R_1$ represents a $C_2$-$C_7$ alkenyl group optionally substituted by one or more groups selected from among the hydroxyl, amine, aryl, $C_1$-$C_4$ alkoxy and $C_1$-$C_4$ aminoalkyl groups, in which case $R_2$ represents a $C_3$-$C_7$ alkenyl group substituted by one more $C_1$-$C_7$ alkoxy groups, or a $C_2$-$C_{10}$ alkynyl group, the latter optionally being substituted by a heteroaryl group;
- or $R_1$ represents:

• a $C_2$-$C_7$ alkenyl group having at least one substituent selected from among oxygen, the halogens and the carboxyl, $C_2$-$C_8$ alkyloxycarbonyl, nitrile, phenoxy, $C_3$-$C_6$ cycloalkyl, heteroaryloxy, $C_1$-$C_7$ alkylsulphonyl, $C_1$-$C_7$ thioalkyl, $C_5$-$C_7$ alkoxy and $C_5$-$C_7$ aminoalkyl groups; a $C_3$-$C_7$ alkenyl group substituted by a heteroaryl group; or

• a $C_2$-$C_7$ alkynyl group having at least one substituent selected from among oxygen, the halogens and the hydroxyl, formyl, carboxyl, $C_2$-$C_8$ alkyloxycarbonyl, nitrile, heteroaryl, $C_1$-$C_7$ alkylsulphonyl, $C_1$-$C_7$ thioalkyl and $C_1$-$C_7$ aminoalkyl groups; or

• a $C_2$-$C_{15}$ alkenyl or alkynyl group substituted by at least one $C_1$-$C_7$ trialkylsilyl group;

in which case $R_2$ represents a hydrogen atom, a hydroxyl group or a $C_l$-$C_7$ alkyl group.

**11.** Quinoline according to any one of claims 8 to 10, **characterised in that** $R_1$ denotes a $C_2$-$C_7$ alkenyl or alkynyl group substituted by one or more halogen atoms, particularly chlorine, bromine or fluorine.

**12.** Quinoline, **characterised in that** it corresponds to general formula (I) according to claim 1 wherein:

- either $R_1$ represents a cyclopropyl-hydroxymethyl, 4-chloro-but-3-en-1-yn-1-yl, hept-1-en-1-y1, 2-bromo-ethenyl, 2-bromoethynyl, 2-bromo-2-fluoro-ethenyl, 4-methylcarboxylate-but 1,3-dien-1-yl, dec-1-yn-1-yl, 2-(2-quinoleyl)-ethenyl, 2-(trimethylsilyl-ethynyl)-4trimethylsilyl-but-1-en-3-yn-1-yl group, in which case $R_2$ denotes a hydrogen atom;
- or $R_1$ represents a prop-1-en-1-yl group, in which case $R_2$ represents a methyl group in the 6 position or a hydroxyl group in the 8 position of the quinoline ring;
- or $R_1$ represents a 2-hydroxypropyl group, in which case $R_2$ represents a hydroxyl group in the 8 position of

the quinoline ring;
- or $R_1$ represents a 2-methylcarboxylate-ethenyl group, in which case $R_2$ represents a methyl group in the 6 position of the quinoline ring.

13. Pharmaceutical composition, **characterised in that** it contains, as active ingredient, at least one quinoline according to claim 12.

**Patentansprüche**

1. Verwendung wenigstens eines Chinolins, entsprechend der allgemeinen Formel (1):

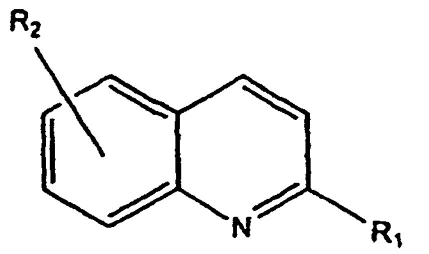

(I)

worin $R_1$
ein Wasserstoff-Atom, eine $C_1$-$C_{15}$-Alkyl-Gruppe, eine $C_2$ $C_{15}$ Alkenyl-Gruppe, eine $C_2$-$C_{15}$-Alkinyl-Gruppe, eine Formyl-Gruppe oder eine Heteroaryl-Gruppe bedeutet, wobei die Letztere gegebenenfalls mit einer oder mehreren Hydroxyl-Gruppen substituiert ist; oder
eine $C_1$-$C_{15}$-Alkyl-Gruppe oder $C_2$-$C_7$-Alkenyl-Gruppe, die wenigstens einen Substituenten trägt, der ausgewählt ist aus Sauerstoff, Halogenen und den Gruppen Hydroxyl, Formyl, Carboxyl, Aryloxycarbonyl, $C_2$-$C_8$ Alkyloxycarbonyl, $C_3$ $C_9$ Alkenyloxycarbonyl, Nitril, Amin, $C_1$-$C_7$-Alkoxy, Phenoxy, $C_3$-$C_6$-Cycloalkyl, Aryl, Heteroaryl, Heteroaryloxy, Arylsulfon, $C_1$-$C_7$-Alkyl-sulfon, $C_1$-$C_7$-Thioalkyl und $C_1$-$C_7$-Aminoalkyl; oder
eine $C_2$-$C_7$-Alkinyl-Gruppe, die wenigstens einen Substituenten trägt, der ausgewählt ist aus Sauerstoff, Halogenen und den Gruppen Hydroxyl, Formyl, Carboxyl, Aryloxycarbonyl, $C_2$-$C_8$-Alkyloxycarbonyl, $C_3$-$C_9$-Alkenyloxycarbonyl, Nitril, Aryl, Heteroaryl, Arylsulfon, $C_1$-$C_7$-Alkylsulfon, $C_1$-$C_7$-Thioalkyl und $C_1$-$C_7$-Aminoalkyl; oder
eine $C_2$-$C_{15}$-Alkenyl- oder $C_2$-$C_{15}$-Alkinyl-Gruppe, die mit wenigstens einer $C_1$-$C_7$-Trialkylsilyl-Gruppe substituiert ist; während
$R_2$, das sich an Position 3, 6 oder 8 am Chinolin-Ring befinden kann, ein Wasserstoff-Atom oder Halogen-Atom bedeutet, eine Hydroxyl-, Formyl-, Carboxyl-, $C_1$-$C_7$-Alkyl-, $C_1$-$C_7$-Alkoxy-, Amin-, $C_1$-$C_{10}$-Alkylamid-, $C_{2}$-$C_7$-Alkenyl-Gruppe, die gegebenenfalls mit einer oder mehreren $C_1$-$C_7$-AlkoxyGruppen substituiert ist, oder auch eine $C_2$-$C_{10}$-Alkinyl-Gruppe, wobei die Letztere gegebenenfalls mit einer Heteroaryl-Gruppe substituiert ist;
oder eines pharmazeutisch annehmbaren Salzes desselben, mit der Maßgabe, dass $R_1$ und $R_2$ nicht beide Wasserstoff-Atome sind, zur Herstellung eines Medikaments für die Behandlung von Coinfektionen mit Protozoen und Retroviren.

2. Verwendung nach Anspruch 1, wobei $R_1$ kein Wasserstoff-Atom ist.

3. Verwendung nach Anspruch 1 oder 2, wobei $R_1$ eine Alkenyl- oder Alkinyl-Gruppe ist, die Substituenten trägt oder nicht.

4. Verwendung nach einem der vorstehenden Ansprüche, wobei $R_1$
eine $C_2$-$C_{15}$-Alkenyl- oder $C_2$-$C_{15}$-Alkinyl-Gruppe bedeutet; oder
eine $C_2$-$C_7$-Alkenyl-Gruppe, die wenigstens einen Substituenten trägt, der ausgewählt ist aus Sauerstoff, Halogenen und den Gruppen Hydroxyl, Formyl, Carboxyl, $C_2$-$C_8$-Alkyloxycarbonyl, Nitril, Amin, $C_1$-$C_7$-Alkoxy, Phenoxy, $C_3$-$C_6$-Cycloalkyl, Aryl, Heteroaryl, Heteroaryloxy, Arylsulfon, $C_1$-$C_7$-Alkylsulfon, $C_1$-$C_7$-Thioalkyl, $C_1$-$C_7$-Aminoalkyl und Trimethylsilyl; oder
eine $C_2$-$C_7$-Alkinyl-Gruppe, die wenigstens einen Substituenten trägt, der ausgewählt ist aus Sauerstoff, Halogenen

und den Gruppen Hydroxyl, Formyl, Carboxyl, $C_2$-$C_8$-Alkyloxycarbonyl, Nitril, Aryl, Heteroaryl, Arylsulfon, $C_1$-$C_7$-Alkylsulfon, $C_1$-$C_7$-Thioalkyl, $C_1$-$C_7$-Aminoalkyl und Trimethylsilyl; während
$R_2$ ein Wasserstoff-Atom, eine Hydroxyl-Gruppe oder eine $C_1$-$C_7$-AlkylGruppe bedeutet.

**5.** Verwendung nach einem der vorstehenden Ansprüche, wobei $R_1$ eine $C_2$-$C_7$-Alkenyl- oder $C_2$-$C_7$-Alkinyl-Gruppe bedeutet, die mit einem oder mehreren Halogen-Atomen, insbesondere Chlor, Brom oder Fluor, substituiert ist.

**6.** Verwendung nach einem der vorstehenden Ansprüche, wobei das Medikament für die Behandlung einer Coinfektion vorgesehen ist, die durch ein oder mehrere Protozoen, die zu den Gattungen *Leishmania, Trypanosoma, Plasmodium, Toxoplasma, Pneumocystis* und *Schistosomia* gehören, sowie durch ein Retrovirus des Typs HIV oder HTLV-1 hervorgerufen wird.

**7.** Verwendung nach Anspruch 6, wobei das Medikament für die Behandlung einer Leishmania/HIV-Coinfektion vorgesehen ist.

**8.** Chinolin mit der allgemeinen Formel (I) nach Anspruch 1, **dadurch gekennzeichnet, dass**
$R_1$ entweder eine $C_1$-$C_7$-Alkyl-Gruppe, eine $C_2$-$C_7$-Alkenyl-Gruppe bedeutet oder eine $C_2$-$C_7$-Alkenyl-Gruppe, die wenigstens einen Substituenten trägt, der ausgewählt ist aus Aryl-Gruppen, wobei $R_2$, das sich an Position 3, 6 oder 8 am Chinolin-Ring befinden kann, eine $C_3$-$C_7$-Alkenyl-Gruppe bedeutet, die mit einer oder mehreren $C_1$-$C_7$-Alkoxy-Gruppen substituiert ist, oder eine $C_2$-$C_{10}$-Alkinyl-Gruppe, die mit einer Heteroaryl-Gruppe substituiert ist;
oder $R_1$ eine $C_1$-$C_7$-Alkyl-Gruppe bedeutet, die wenigstens einen Substituenten trägt, der ausgewählt ist aus den Gruppen Hydroxyl, Amin, $C_1$-$C_4$-Alkoxy und $C_1$-$C_4$-Aminoalkyl, oder $R_1$ eine $C_2$-$C_7$-Alkenyl-Gruppe bedeutet, die wenigstens einen Substituenten trägt, der ausgewählt ist aus den Gruppen Hydroxyl, Amin, $C_1$-$C_4$-Alkoxy und $C_1$-$C_4$-Aminoalkyl, wobei $R_2$, das sich an Position 3, 6 oder 8 am Chinolin-Ring befinden kann, eine $C_3$-$C_7$-Alkenyl-Gruppe bedeutet, die mit einer oder mehreren $C_1$-$C_7$-AlkoxyGruppen substituiert ist, oder eine $C_2$-$C_{10}$-Alkinyl-Gruppe, wobei die Letztere gegebenenfalls mit einer Heteroaryl-Gruppe substituiert ist;
oder $R_1$ eine Methyl- oder Ethyl-Gruppe bedeutet, die wenigstens einen Substituenten trägt, der ausgewählt ist aus Halogenen;
wobei $R_2$, das sich an Position 3, 6 oder 8 am Chinolin-Ring befinden kann, eine $C_1$-$C_7$-Alkoxy-, Amin-, $C_1$-$C_{10}$-Alkylamid-, $C_2$-$C_7$-Alkenyl-Gruppe bedeutet, die mit einer oder mehreren $C_1$-$C_7$-Alkoxy-Gruppen substituiert ist, oder auch eine $C_2$-$C_{10}$-Alkinyl-Gruppe, die mit einer Heteroaryl-Gruppe substituiert ist;
oder $R_1$ für einen 2-Pyridyl-Rest steht,
wobei $R_2$, das sich an Position 3, 6 oder 8 am Chinolin-Ring befinden kann, ein Wasserstoff-Atom oder Halogen-Atom bedeutet, Formyl, Carboxyl, $C_1$-$C_7$-Alkyl, $C_1$-$C_7$-Alkoxy, Amin, $C_1$-$C_{10}$-Alkylamid, $C_2$-$C_7$-Alkenyl, das gegebenenfalls mit einer oder mehreren $C_1$-$C_7$-Alkoxy-Gruppen substituiert ist, oder auch eine $C_2$-$C_{10}$-Alkinyl-Gruppe, wobei die Letztere gegebenenfalls mit einer Heteroaryl-Gruppe substituiert ist;
oder $R_1$ eine $C_8$-$C_{15}$-Alkyl-Gruppe bedeutet, die gegebenenfalls einen Substituenten trägt, der ausgewählt ist aus Aryl-Gruppen; oder
eine $C_8$- $C_{15}$ Alkenyl-Gruppe, eine $C_2$-$C_7$-Alkenyl-Gruppe, die wenigstens einen Substituenten trägt, der ausgewählt ist aus Arylsulfonen; oder
eine $C_2$-$C_{15}$-Alkinyl-Gruppe, eine $C_2$-$C_7$-Alkinyl-Gruppe, die wenigstens einen Substituenten trägt, der ausgewählt ist aus Aryl- und Arylsulfon-Gruppen;
wobei $R_2$, das sich an Position 3, 6 oder 8 am Chinolin-Ring befinden kann, für eine Gruppe steht, die ausgewählt ist aus $C_1$-$C_7$-Alkoxy, Amin, $C_1$-$C_{10}$-Alkylamid, $C_2$-$C_7$-Alkenyl, das gegebenenfalls mit einer oder mehreren $C_,$-$C_7$-Alkoxy-Gruppen substituiert ist, oder auch eine $C_2$-$C_{10}$-Alkinyl-Gruppe, die mit einer Heteroaryl-Gruppe substituiert ist;
oder $R_1$ ein Wasserstoff-Atom, eine Formyl-Gruppe, eine Heteroaryl-Gruppe bedeutet, die gegebenenfalls mit einer oder mehreren Hydroxyl-Gruppen substituiert ist, wobei der 2-Pyridyl-Rest ausgenommen ist; oder
eine $C_1$-$C_{15}$-Alkyl-Gruppe, die wenigstens einen Substituenten trägt, der ausgewählt ist aus Sauerstoff und den Gruppen Formyl, Carboxyl, Aryloxycarbonyl, $C_2$-$C_8$-Alkyloxycarbonyl, $C_3$-$C_9$-Alkenyloxycarbonyl, Nitril, Phenoxy, $C_3$-$C_6$-Cycloalkyl, Heteroaryloxy, Arylsulfon, $C_1$-$C_7$-Alkylsulfon und $C_1$-$C_7$-Thioalkyl, eine $C_1$-$C_7$-Alkyl-Gruppe, die wenigstens einen Substituenten trägt, der ausgewählt ist aus den Gruppen $C_5$-$C_7$-Alkoxy und $C_5$-$C_7$-Aminoalkyl, eine $C_3$-$C_{15}$-Alkyl-Gruppe, die wenigstens einen Substituenten trägt, der ausgewählt ist aus Halogenen, eine $C_6$-$C_{15}$-Alkyl-Gruppe, die mit wenigstens einer Heteroaryl-Gruppe substituiert ist, eine $C_8$-$C_{15}$-Alkyl-Gruppe, die wenigstens einen Substituenten trägt, der ausgewählt ist aus den Gruppen Hydroxyl, Amin, $C_1$-$C_7$-Alkoxy und $C_1$-$C_7$-Aminoalkyl; oder eine $C_2$-$C_7$-Alkenyl-Gruppe, die wenigstens einen Substituenten trägt, der ausgewählt ist aus Sauerstoff, Halogenen und den Gruppen Carboxyl, Aryloxycarbonyl, $C_2$-$C_8$-Alkyloxycarbonyl, $C_3$-$C_9$-Alkenyloxycarbonyl, Nitril, Phenoxy, $C_3$-$C_6$-Cycloalkyl, Heteroaryloxy, $C_1$-$C_7$-Alkylsulfon, $C_1$-$C_7$-Thioalkyl, $C_1$-$C_7$-Alkoxy und $C_5$-$C_7$-Aminoalkyl; eine $C_3$-$C_7$-Alkenyl-Gruppe, die mit einer Heteroaryl-Gruppe substituiert ist; oder

eine $C_2$-$C_7$-Alkinyl-Gruppe, die wenigstens einen Substituenten trägt, der ausgewählt ist aus Sauerstoff, Halogenen und den Gruppen Hydroxyl, Formyl, Carboxyl, Aryloxycarbonyl, $C_2$-$C_8$-Alkyloxycarbonyl, $C_3$-$C_9$-Alkenyloxycarbonyl, Nitril, Heteroaryl, $C_1$-$C_7$-Alkylsulfon, $C_1$-$C_7$-Thioalkyl und $C_1$-$C_7$-Aminoalkyl; oder
eine $C_2$-$C_{15}$-Alkenyl- oder $C_2$-$C_{15}$-Alkinyl-Gruppe, die mit wenigstens einer $C_1$-$C_7$ Trialkylsilyl-Gruppe substituiert ist;
wobei $R_2$, das sich an Position 3, 6 oder 8 am Chinolin-Ring befinden kann, ein Wasserstoff-Atom oder Halogen-Atom bedeutet, eine Hydroxyl-, Formyl-, Carboxyl-, $C_1$-$C_7$-Alkyl-, $C_1$-$C_7$-Alkoxy-, Amin-, $C_1$-$C_{10}$-Alkylamid-, $C_2$-$C_7$-Alkenyl-Gruppe, die gegebenenfalls mit einer oder mehreren $C_1$-$C_7$-Alkoxy-Gruppen substituiert ist, oder auch eine $C_2$ $C_{10}$-Alkinyl-Gruppe, wobei die Letztere gegebenenfalls mit einer Heteroaryl-Gruppe substituiert ist; oder ein pharmazeutisch annehmbares Salz desselben, mit der Maßgabe, dass $R_1$ und $R_2$ nicht beide Wasserstoff-Atome sind, zur Verwendung als Medikament.

**9.** Chinolin nach Anspruch 8, **dadurch gekennzeichnet, dass** $R_1$ eine Alkenyl-oder Alkinyl-Gruppe bedeutet, die Substituenten trägt oder nicht.

**10.** Chinolin nach Anspruch 8 oder 9, **dadurch gekennzeichnet, dass**
$R_1$ entweder eine $C_2$-$C_7$-Alkenyl-Gruppe bedeutet, die gegebenenfalls mit einer oder mehreren Gruppen substituiert ist, die ausgewählt sind aus den Gruppen Hydroxyl, Amin, Aryl, $C_1$-$C_4$-Alkyloxy und $C_1$-$C_4$-Aminoalkyl, wobei $R_2$ eine $C_3$-$C_7$-Alkenyl-Gruppe bedeutet, die mit einer oder mehreren $C_1$-$C_7$-Alkoxy-Gruppen substituiert ist, oder eine $C_2$-$C_{10}$-Alkinyl-Gruppe, wobei die Letztere gegebenenfalls mit einer Heteroaryl-Gruppe substituiert ist;
oder $R_1$ eine $C_2$-$C_7$-Alkenyl-Gruppe bedeutet, die wenigstens einen Substituenten trägt, der ausgewählt ist aus Sauerstoff, Halogenen und den Gruppen Carboxyl, $C_2$-$C_8$-Alkyloxycarbonyl, Nitril, Phenoxy, $C_3$-$C_6$-Cycloalkyl, Heteroaryloxy, $C_1$-$C_7$-Alkylsulfon, $C_1$-$C_7$- Thioalkyl, $C_5$-$C_7$-Alkoxy und $C_5$-$C_7$-Aminoalkyl; eine $C_3$-$C_7$-Alkenyl-Gruppe, die mit einer Heteroaryl-Gruppe substituiert ist; oder
eine $C_2$-$C_7$-Alkinyl-Gruppe, die wenigstens einen Substituenten trägt, der ausgewählt ist aus Sauerstoff, Halogenen und den Gruppen , Hydroxyl, Formyl, Carboxyl, $C_2$-$C_8$-Alkyloxycarbonyl, Nitril, Heteroaryl, $C_1$-$C_7$-Alkylsulfon, $C_1$-$C_7$-Thioalkyl und $C_1$-$C_7$-Aminoalkyl, oder
eine $C_2$-$C_{15}$-Alkenyl- oder $C_2$-$C_{15}$-Alkinyl-Gruppe, die mit wenigstens einer $C_1$-$C_7$- Trialkylsilyl-Gruppe substituiert ist;
wobei $R_2$ ein Wasserstoff-Atom, eine Hydroxyl-Gruppe oder eine $C_1$-$C_7$-Alkyl-Gruppe bedeutet.

**11.** Chinolin nach einem der Ansprüche 8 bis 10, **dadurch gekennzeichnet, dass** $R_1$ eine $C_2$-$C_7$-Alkenyl- oder $C_2$-$C_7$-Alkinyl-Gruppe bedeutet, die mit einem oder mehreren Halogen-Atomen, insbesondere Chlor, Brom oder Fluor, substituiert ist.

**12.** Chinolin, **dadurch gekennzeichnet, dass** es der allgemeinen Formel (I) nach Anspruch 1 entspricht, worin
$R_1$ entweder eine Cyclopropylhydroxymethyl-Gruppe, eine 4-Chlorbut-3-en-1-in-1-yl-Gruppe, eine Hept-1-en-1-yl-Gruppe, eine 2-Bromethenyl-Gruppe, eine 2-Bromethinyl-Gruppe, eine 2-Brom-2-fluorethenyl-Gruppe, eine 4-Methoxycarbonylbuta-1,3-dien-1-yl-Gruppe, eine Dec-1-in-1-yl-Gruppe, eine 2-(2-Chinolyl)ethenyl-Gruppe, eine 2-(Trimethylsilylethinyl)-4-trimethylsilylbut-1-en-3-in-1-yl-Gruppe bedeutet, wobei $R_2$ für ein Wasserstoff-Atom steht;
oder $R_1$ eine Prop-1-en-1-yl-Gruppe bedeutet, wobei $R_2$ eine Methyl-Gruppe in Position 6 oder eine Hydroxyl-Gruppe in Position 8 des Chinolin-Rings bedeutet;
oder $R_1$ eine 2-Hydroxypropyl-Gruppe bedeutet, wobei $R_2$ eine Hydroxyl-Gruppe in Position 8 des Chinolin-Rings bedeutet;
oder $R_1$ eine 2-Methoxycarbonylethenyl-Gruppe bedeutet, wobei $R_2$ eine Methyl-Gruppe in Position 6 des Chinolin-Rings bedeutet.

**13.** Pharmazeutische Zusammensetzung, **dadurch gekennzeichnet, dass** sie als Wirkstoff wenigstens ein Chinolin nach Anspruch 12 umfasst.

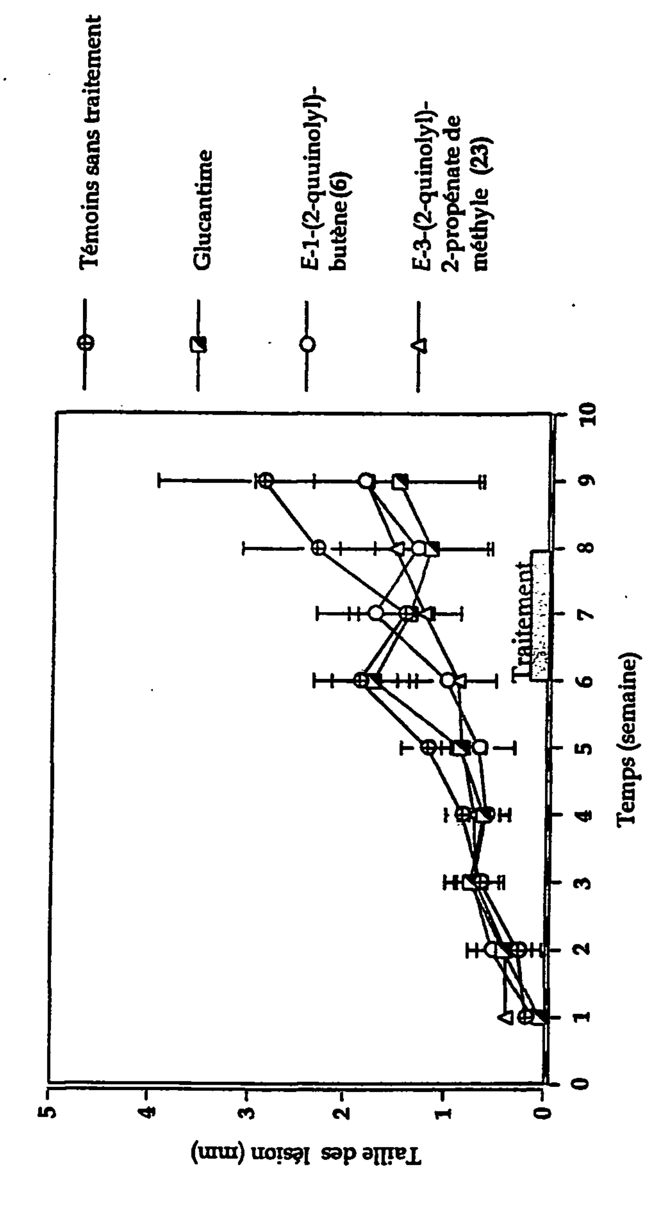
Témoins sans traitement
Glucantime
E-1-(2-quinolyl)-butène (6)
E-3-(2-quinolyl)-2-propénate de méthyle (23)
Traitement
Temps (semaine)
Taille des lésion (mm)
0
1
2
3
4
5
6
7
8
9
10

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9307125 A
- WO 9845269 A


### Littérature non-brevet citée dans la description


- **OMS.** *Weekly Epidemiol. Rec.,* 1997, vol. 72, 49-54
- **WOLDAY et al.** *Parasitology Today,* 1999, vol. 15, 182-187
- **BERNIER et al.** *J. Virol.,* 1995, vol. 69, 7282-7275
- **WOLDAY et al.** *Scand J. Infect. Dis.,* 1998, vol. 30, 29-34
- *Journal of Medicinal Chemistry,* 2000, vol. 43, 1533-1540
- **WEBB.** *Tetrahedron Lett.,* 1985, vol. 26, 3191-3194
- **FAKHFAKH et al.** *Tetrahedron Lett.,* 2001, vol. 42, 3847-3850
- **FAKHFAKH et al.** *J. Organomet. Chem.,* 2001, vol. 624, 131-135
- **ANTOINE et al.** *Parasitology,* 1989, vol. 99, 1